# EUROPÄISCHE PATENTSCHRIFT

(11) **EP 0 701 628 B1**
(45) Veröffentlichungstag und Bekanntmachung des Hinweises auf die Patenterteilung: **21.01.1998**
(21) Anmeldenummer: 94918807.2
(22) Anmeldetag: 27.05.1994
(51) Int. Cl.: C12Q 1/68, C12N 15/85

(54) **VERFAHREN ZUM SCREENEN VON SUBSTANZEN MIT MODULIERENDER WIRKUNG AUF EINEN INTERLEUKIN-5-REZEPTOR-ABHÄNGIGEN ZELLULÄREN SIGNALÜBERTRAGUNGSWEG**
PROCESS FOR SCREENING SUBSTANCES HAVING A MODULATING EFFECT ON AN INTERLEUKINE-5-RECEPTOR-DEPENDENT CELLULAR SIGNAL TRANSMISSION PATH
PROCEDE DE CRIBLAGE DE SUBSTANCES A EFFET MODULATEUR SUR LA VOIE DE TRANSMISSION CELLULAIRE DE SIGNAUX DEPENDANT D'UN RECEPTEUR A L'INTERLEUKINE 5

(30) Priorität: 27.05.1993 DE 4317577
(43) Veröffentlichungstag der Anmeldung: 20.03.1996
(73) Patentinhaber: BOEHRINGER INGELHEIM INTERNATIONAL GmbH, 55218 Ingelheim am Rhein (DE)
(72) Erfinder: CZERNILOFSKY, Armin, Peter, A-1130 Wien (AT); WEYER, Ulrike, A-1130 Wien (AT); YOUNG, Ian, G., Hawker, ACT 2614 (AU)
(86) Internationale Anmeldenummer: EP9401735
(87) Internationale Veröffentlichungsnummer: WO9428170

(56) Entgegenhaltungen:
- WO-A-92/02639
- WO-A-92/13063
- J. RECEPTOR RESEARCH, Bd.13, Nr.1-4, 1993 HIMMLER ET AL. 'Functional testing of human dopamine D1 and D5 receptors expressed in stable cAMP-responsive luciferase reporter cell lines' in der Anmeldung erwähnt
- CELL, Bd.61, Mai 1990, NEW YORK US Seiten 485 - 496 LAMB ET AL. 'Demonstration in living cells of an intragenic negative regulatory element within the rodent c-fos gene' in der Anmeldung erwähnt
- JOURNAL OF IMMUNOLOGY, Bd.147, Nr.8, 15. Oktober 1991, BALTIMORE US Seiten 2777 - 2786 VORABERGER ET AL. 'Cloning of the human gene ICAM-1 and analysis of its 5'-regulatory region' in der Anmeldung erwähnt
- CELL, Bd.54, Juli 1988, NEW YORK US Seiten 325 - 334 SCHÖNTHAL ET AL. 'Requirement for fos gene expression ...' in der Anmeldung erwähnt

### SEQUENZPROTOKOLL

### (1) ALLGEMEINE INFORMATION:

(i) ANMELDER:
   (A) NAME: Boehringer Ingelheim International GmbH
   (B) STRASSE: Postfach 200
   (C) ORT: Ingelheim am Rhein
   (E) LAND: BRD
   (F) POSTLEITZAHL: 55216
   (G) TELEPHON: 06132/772282
   (H) TELEFAX: 06132/774377
(ii) ANMELDETITEL: Verfahren zum Screenen von Substanzen mit modulierender Wirkung auf einen Interleukin-5-Rezeptor-abhaengigen zellulaeren Signaluebertragungsweg
(iii) ANZAHL DER SEQUENZEN: 2
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Realease #1.0, Version #1.25 (EPA)

### (2) INFORMATION ZU SEQ ID NO: 1:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: cDNS
(iii) ANTISENSE: NEIN
(ix) MERKMALE:
   (A) NAME/SCHLÜSSEL: 5'UTR
   (B) LAGE: 1..21
(xi) SEQUENZBESCHREIBUNG: SEQ ID NO: 1:
   TCGGCTACCA TGGTGCCTGT G 21

### (2) INFORMATION ZU SEQ ID NO: 2:

(i) SEQUENZ CHARAKTERISTIKA:
   (A) LÄNGE: 24 Basenpaare
   (B) ART: Nukleinsäure
   (C) STRANGFORM: Einzel
   (D) TOPOLOGIE: linear
(ii) ART DES MOLEKÜLS: cDNS
(iii) ANTISENSE: NEIN
(ix) MERKMALE:
   (A) NAME/SCHLÜSSEL: 5'UTR
   (B) LAGE: 1..24
(xi) SEQUENZBESCHREIBUNG: SEQ ID NO: 2:
   ATGGCAAAAT GCCATCAAAA CGTG 24

## Beschreibung

Verfahren zum Screenen von Substanzen mit modulierender Wirkung auf einen Interleukin-5-Rezeptor-abhängigen zellulären Signalübertragungsweg.

Die vorliegende Erfindung bezieht sich auf ein Verfahren zum Auffinden pharmakologisch wirksamer Substanzen durch Bestimmung der modulierenden Wirkung von Substanzen auf einen Interleukin-5-Rezeptorabhängigen Signalübertragungsweg in menschlichen oder tierischen Zellen.

Mit herkömmlichen sog. "Radioligandentests", in denen eine Substanz daraufhin untersucht wird, inwieweit sie einen an einen Rezeptor gebundenen Liganden verdrängen kann, und die zum Auffinden pharmakologisch wirksamer Substanzen eingesetzt werden, können nur Substanzen identifiziert werden, die die Bindung von bekannten Liganden Rezeptor-Bindungsstellen beeinflussen. Diese Tests können nicht unterscheiden, ob die bindende Substanz agonistische oder antagonistische Wirkung hat.

Transmembran-Signalübertragungssysteme höherer eukaryotischer Zellen bestehen häufig aus den folgenden membrangebundenen Komponenten:
a) einem Zelloberflächenrezeptor, der gegebenenfalls aus mehreren Untereineinheiten bestehen kann; gegebenenfalls
b) einem Guanin-Nukleotid-bindenden und GTP spaltenden regulatorischen Protein, das als G-Protein bezeichnet wird und das sowohl an den Rezeptor als auch an seinen Effektor gekoppelt werden kann;
c) sog. "Effektoren", die oft zur Veränderung der Konzentration sekundärer Botenmoleküle, wie cAMP, DAG, IP₃ etc. führen, z.B. einem Ionenkanal oder Adenylatzyklasen, Guanylatzyklasen oder Phospholipasen; und/oder
d) verschiedenen Kinasen.

Zelloberflächenrezeptoren erkennen die passenden Liganden aus einer Vielzahl von extrazellulären Stimuli. Durch die Bindung des Liganden an den Rezeptor wird eine Signalkaskade aktiviert. Diese beginnt im Falle von G-Protein-gekoppelten Rezeptoren, deren Signalübertragungsweg relativ gut aufgeklärt ist und die die Wirkungen von sehr unterschiedlichen extrazellulären Signalen vermitteln, mit der Aktivierung des heterotrimären G-Proteins.

Niedermolekulare sekundäre Botenstoffe ("Second Messengers"), wie cAMP (zyklisches AMP), ausgelöst durch Aktivierung der Adenylatzyklase, cGMP (zyklisches GMP), ausgelöst durch Aktivierung der Guanylatzyklase, oder Inositol-1,4,5-Triphosphat (IP₃) und Diacylglyzerine (DAG), ausgelöst durch Aktivierung von Phospholipasen, wie Phospholipase C oder, unter Beteiligung von Hydrolasen, Phospholipase D (Billah et al., 1989), bewirken ihrerseits intrazelluläre Veränderungen, u.a. die selektive Phosphorylierung von Proteinen durch Aktivierung von Proteinkinasen (z.B. PKC durch IP₃/DAG, PKA durch cAMP), die Beeinflussung der Regulation der Transkription bestimmter Gene und die Proliferation. (Eine antagonistisch wirksame Substanz kann die durch eine agonistisch wirksame Substanz hervorgerufene Wechselwirkung und die dadurch bewirkte Konzentrationsänderung des Second Messengers ganz oder teilweise aufheben bzw. selbst zu einem reversen funktionellen Effekt führen.) Über dieses Signalübertragungssystem können Zellen miteinander kommunizieren und ihre Entwicklung bzw. die durch sie ausgelösten Wirkungen koordinieren.

Da ein einziger Rezeptor-Subtyp (möglicherweise in derselben Zelle oder in verschiedenen Zellen) an mehr als einen Effektor gekoppelt sein kann und viele Rezeptor-Subtypen dieselben Effektoren aktivieren können, werden komplizierte Signalübertragungsnetzwerke ausgebildet. Die als Folge der Aktivierung des Rezeptors im Zuge der Signalübertragungskaskade aktivierten Transkriptionsfaktoren (z.B. CREB-Protein, AP1-Protein) treten in Wechselwirkung mit regulatorischen DNA-Elementen. Beispiele dafür sind CRE (CRE-Element, "cAMP responsive element") bzw. TRE (TRE = "TPA responsive element"; TPA = Phorbol-12-Myristat-13-Acetat = Phorbolester), die CREB bzw. AP1 binden: viele Gene, deren Transkription durch cAMP reguliert ist (z.B. Ratten-Somatostatin, Human-α-Gonadotropin, c-Fos), enthalten in der 5'-flankierenden Region eine konservierte Sequenz als regulatorisches Element. Die CRE-Sequenz ist identisch oder ähnlich dem palindromischen Octamer TGACGTCA (Montminy et al., 1990). TRE-Elemente enthalten das sehr ähnliche heptamere Motiv TGAGTCA, das sich von der CRE-Element-Konsensussequenz nur durch ein einziges Nukleotid unterscheidet (Deutsch et al., 1988). Das TRE-Motiv, oder sehr ähnliche Motive, wurde in vielen Genen identifiziert, deren Transkription durch Phorbolester aktiviert wird (Angel et al., 1987a und b; Lee et al., 1987). Umgebende DNA-Sequenzen bzw. Protein-Protein-Wechselwirkungen mit anderen Faktoren bestimmen u.a. die konkreten Regulationsphänomene an einem bestimmten Gen.

Weitere regulatorische DNA-Elemente sind sog. "SRE-Elemente", die auf Serumfaktoren ansprechen ("serum responsive element"; Treisman, 1985). SRE-Elemente sind induzierbare cis-Elemente, die aus 20 Basenpaaren mit einer dyadischen symmetrischen Struktur bestehen. SRE-Elemente sprechen auf verschiedene Wachstumsfaktoren an; kürzlich wurde berichtet, daß SRE das maßgebliche Ziel für die c-fos-Induktion durch verschiedene Zytokine ist (Hatakeyama et al., 1989). Die auf die Konzentrationsänderung von Glucocorticoiden ansprechenden Motive werden als "GRE-Elemente" bezeichnet (Yamamoto, 1985; Scheidereit et al., 1986; Jantzen et al., 1987; Strähle et al., 1987).

Bedingt durch die Komplexität des Netzwerkes der Signalübertragungswege kann es zu einem sog. "crosstalk" zwischen Signalübertragungswegen, z.B. dem Adenylatzyklase- und dem Phospholipase C-Signalübertragungsweg, kommen. Unter "crosstalk" wird das Phänomen verstanden, daß die Beeinflussung eines Effektorsystems auch zur Beeinflussung eines anderen führt (Sassone-Corsi et al., 1990; Houslay, 1991). Das Phänomen des Crosstalk wird physiologisch zur Signalintegration bzw. Signalvernetzung verwendet, um eine Redundanz der Signale herzustellen bzw. die Kommunikation der verschiedenen Signalübertragungswege zu gewährleisten. Crosstalk kann auf verschiedenen Ebenen des Signalübertragungsweges stattfinden. Eine mögliche Ursache für pathologische Veränderungen der Zelle ist die Störung dieser Wechselwirkungen, z.B. wenn ein bestimmter Rezeptor im physiologischen Sinn nicht korrekt mit einem Effektorsystem wechselwirkt.

Zu Rezeptoren, die nicht an G-Proteine gekoppelt sind, gehören die Zytokin-Rezeptoren. Von ihnen wird vermutet, daß sie mit mehreren Signaltransduktionswegen gekoppelt sind, die wiederum für verschiedene Rezeptoren überlappen, so daß innerhalb der Zelle ein multipler crosstalk erfolgt.

Zytokine spielen eine wichtige Rolle bei der Koordination von Immun- und Entzündungsreaktionen (Lymphokine und Monokine), bei der Abwehr viraler Infektionen (Interferone) und bei der Hämatopoese ("colony-stimulating factors", CSF). Sie regulieren Proliferation, Differenzierung und Funktion einer Vielzahl von Zellen innerhalb hämatopoetischer, lymphoider oder anderer Organsysteme. Jedes einzelne Zytokin übt seine Wirkung auf verschiedene Zelltypen aus, und einzelne Zellen reagieren oft auf mehrere Zytokine, so daß Zytokin-produzierende Zellen und Zellen, die durch Zytokine reguliert werden, ein komplexes interzelluläres Netzwerk bilden, bei dem die Zytokine synergistisch oder entgegengesetzt wirken. Das Spektrum der biologischen Aktivität eines Zytokins wird u.a. bedingt durch die Anzahl von verschiedenen Zelltypen, die spezifische Rezeptoren für das Zytokin exprimieren. Interleukin-3 (IL-3) und Granulocyte-macrophage-colony-stimulating-factor (GM-CSF) sind Beispiele für pleiotrope Faktoren, die multipotente hämatopoetische Zellen sowie Vorläuferzellen für eine ganze Reihe von hämatopoetischen Linien stimulieren. Interleukin-5 (IL-5) dagegen hat eine weitaus geringere Wirkungsbreite, es wirkt vor allem auf eosinophile Vorläuferzellen (Miyajima et al., 1992).

Die Analyse ihrer Proteinstrukturen hat gezeigt, daß alle Zytokin-Rezeptoren membrangebundene Glykoproteine sind, die aus einer extrazellulären N-terminalen Domäne, einer einzelnen Transmembran-Domäne und einer zytoplasmatischen Domäne bestehen. Die extrazellulären Domänen weisen eine Reihe von gemeinsamen strukturellen Komponenten auf, nach denen die Zytokin-Rezeptoren mehreren Rezeptorgenfamilien zugeordnet werden können, deren Mitglieder vermutlich von gemeinsamen Vorläufergenen abstammen (Gillis, 1991). Das IL-5-Rezeptorgen gehört zur Hämatopoetin-Rezeptorgenfamilie. Die Mitglieder dieser Familie enthalten charakteristische konservierte Cysteinreste, die einzeln oder in multiplen Kopien in der extrazellulären Domäne vorkommen. Außerdem enthalten sie ein Tryptophan-Serin-X-Tryptophan-Serin-Motiv (wobei X jede beliebige Aminosäure sein kann), das häufig in der extrazellulären Domäne nahe der Transmembran-Domäne lokalisiert ist. Schließlich weisen Rezeptoren dieser Familie einzelne oder multiple Kopien des Fibronektin-Typ 3-Bindungs-Motivs, verstreut über die gesamte extrazelluläre Domäne, auf (Gillis, 1991).

Zu der Hämatopoetin-Rezeptor-Familie gehören außer dem IL-5-Rezeptor (beide Untereinheiten) auch beide Untereinheiten der Rezeptoren für IL-3, IL-6 und GM-CSF, IL-4, IL-7, Erythropoetin (EPO) und "leukemia inhibitory factor" (LIF)-Rezeptoren, die β-Untereinheit des IL-2 Rezeptors sowie die Rezeptoren für "growth hormone" (GH), Prolaktin und G-CSF.

Der IL-5-Rezeptor besteht aus einer α-Untereinheit, die die spezifische Zytokin-Bindungsdomäne enthält, und einer β-Untereinheit, die zur Ausbildung einer hohen Affinität des Rezeptors für IL-5 beiträgt und für die Signaltransduktion notwendig ist. Die für die humane (Tavernier et al., 1991; Murata et al., 1992) und die murine (Takaki et al., 1990) α-Untereinheit, sowie die humane (Tavernier et al., 1991) und die murine (Devos et al., 1991; Takaki et al., 1991) β-Untereinheit des IL-5-Rezeptors kodierenden DNAs wurden kloniert. Die Rezeptoren für IL-5, IL-3 und GM-CSF haben eine identische β-Untereinheit (Kitamura et al., 1991; Tavernier et al., 1991). Wenn mehr als einer der drei Rezeptoren auf einer Zelle exprimiert wird, kann es daher auf Rezeptorebene zu einem "cross-talk" zwischen den Zytokinen kommen.

Die Untereinheiten der Zytokin-Rezeptoren enthalten im Gegensatz zu Rezeptoren für Wachstumsfaktoren wie EGF oder PDGF keine intrinsische Tyrosin Kinase-Aktivität. Es wurde jedoch gezeigt, daß die Stimulation des IL-5-, des IL-3- bzw. des GM-CSF Rezeptors durch den jeweiligen Liganden eine schnelle Tyrosin-Phosphorylierung bestimmter zellulärer Proteine induziert (Koyasu et al., 1987; Isfort et al., 1988; Morla et al., 1988; Murata et al., 1990). Es wurde eine Tyrosinkinase identifiziert, die nach Stimulation mit GM-CSF mit der β-Untereinheit des Rezeptors assoziiert (Hanazono et al., 1993). Weitere Vorgänge, die bei der GM-CSF-induzierten Signalübertragung ablaufen, sind die Aktivierung von p21^{ras} (Satoh et al., 1991, 1992) und von c-raf (Carroll et al., 1990), während für die IL-3-induzierte Signaltransduktion außer einer Aktivierung von p21^{ras} (Satoh et al., 1991, 1992) und c-raf (Carroll et al., 1990) auch eine Aktivierung von Proteinkinase C (Whetton et al., 1988) und eine Phosphorylierung von "mitogen-activated protein" (MAP)-Kinasen (Welham and Schrader, 1992) gezeigt wurde. Die genaue Reihenfolge dieser Vorgänge innerhalb der gesamten Signaltransduktionskaskaden ist jedoch bisher unbekannt.

Die Gene, die nach Stimulation von Zytokin-Rezeptoren über Komponenten der Signaltransduktionsmaschinerie induziert werden, sind weitgehend noch nicht identifiziert. Damit ist auch über die regulatorischen Elemente in den Promotoren solcher Gene wenig bekannt. Beispielsweise führt die Stimulation IL-6 Rezeptors zur Aktivierung eines spezifischen DNA-Bindungsproteins, NF-IL-6, das durch Bindung an IL-6 responsive Elemente im Promotorbereich von Zielgenen die Expression der Gene bewirkt und damit IL-6- abhängige biologische Effekte vermittelt (Akira et al., 1990). Das c-fos Proto-Onkogen, das multiple induzierbare Sequenzelemente in der Promotorregion enthält (Verma und Sassone-Corsi, 1987), wird durch IL-2, IL-3 und EPO induziert, wobei an seiner Induktion u.a. das im c-fos Promotor vorhandene "serum-response-element" (SRE) beteiligt ist (Hatakeyama et al., 1989, 1992).

Der IL-5-Rezeptor wird durch Bindung seines Liganden Interleukin-5 (IL-5) aktiviert. IL-5 ist ein Peptidhormon, das von T-Zellen und Mastzellen produziert wird und eine wesentliche Funktion bei der Enddifferenzierung der Eosinophilen hat.

Über die Signalübertragungswege, die an den IL-5-Rezeptor gekoppelt sind, ist fast nichts bekannt. Außer der Phosphorylierung einiger, bisher nicht identifizierter zellulärer Proteine (Murata et al., 1990) sind keine Vorgänge bei der durch Stimulation des IL-5-Rezeptors ausgelösten Signaltransduktion aufgeklärt worden. Da die drei Zytokine IL-5, IL-3 und GM-CSF unterschiedliche biologische Funktionen haben, ist es möglich, daß außer der β-Untereinheit, die allen drei Rezeptoren gemeinsam ist, auch die zytokinspezifischen α-Untereinheiten der Rezeptoren eine Rolle bei der Induktion von intrazellulären Signalwegen spielen, insbesondere von zytokinspezifischen Signalübertragungen und Funktionen (Tominaga et al., 1992). Es lassen sich daher aus den Kenntnissen über die durch IL-3 Rezeptor- bzw. GM-CSF Rezeptor-Aktivierung ausgelösten Signalübertragungsvorgänge keine eindeutigen Rückschlüsse auf die an den IL-5-Rezeptor gekoppelten Signalwege ziehen. Da auch noch keine Gene identifiziert wurden, die nach Stimulation des IL-5-Rezeptors induziert werden, ist auch nichts bekannt über regulatorische Elemente, die in der Promotorregion solcher Gene vorhanden sind und die auf durch Aktivierung des IL-5-Rezeptors in Gang gesetzte Signalübertragungswege ansprechen.

Die Aktivierung des IL-5-Rezeptors durch Bindung seines Liganden wurde in Zusammenhang mit verschiedenen Erkrankungen gebracht, die mit einer Eosinophilie, also einer Vermehrung der Eosinophilen im Blut, einhergehen. Dazu zählen Autoimmunerkrankungen, Transplantatabstoßungen, allergische Erkrankungen, bestimmte parasitäre Erkrankungen und asthmatische Erkrankungen. Bisher für eine kausale Therapie derartiger Erkrankungen vorgeschlagene Ansätze bestehen einerseits in der Verabreichung der löslichen α-Untereinheit des IL-5-Rezeptors als Antagonist (EP-A 0 492 214), andererseits, ausgehend von der Kristallstuktur von IL-5, in einem rationalen Design von Antagonisten oder Agonisten (Milburn et al., 1993).

Aufgrund des Zusammenhanges zwischen der Aktivierung des IL-5-Rezeptors und phathologischen Zuständen besteht Bedarf an einem Screeningverfahren, mit dem Arzneimittel zur Behandlung dieser Erkrankungen identifiziert werden können.

In der WO 92/13063 wird ein Screeningverfahren zum Auffinden von Substanzen beschrieben, welche die Transkription eines Gens direkt modulieren, indem sie auf der Ebene des Transkriptionsfaktors wirken. Die eingesetzten Testzellen enthalten ein Konstrukt, bei dem das Reportergen vom Promotor desjenigen Gens kontrolliert wird, dessen Expression beeinflußt werden soll.

In jüngster Zeit wurden Screening-Verfahren entwickelt, die es ermöglichen, für die Behandlung von pathologischen Zuständen Arzneimittel zu finden, die spezifisch für einen bestimmten Rezeptor bzw. Rezeptorsubtyp sind und die darüberhinaus spezifisch den rezeptorabhängigen Signalübertragungsweg beeinflussen. Diese Verfahren beruhen auf dem Prinzip, daß die modulierende Wirkung von Substanzen auf den rezeptorabhängigen Signalübertragungsweg über die Expression von Genen in Testzellen nachgewiesen werden kann:

Ein Beispiel dafür ist das Verfahren gemäß der WO 92/02639, in dem die Testzellen ein Reportergenkonstrukt enthalten, das auf die Konzentrationsänderung eines Botenstoffes des zu beeinflussenden Signalübertragungsweges anspricht.

Ein von King et al., 1990, beschriebenes Assay-System beruht auf der Beeinflussung des Signalübertragungsweges, der von den G-Proteingekoppelten Pheromon-Rezeptoren von *Saccharomyces cerevisiae* benutzt wird, wobei in der Hefezelle die Reaktion auf die Bindung einer agonistisch wirksamen Verbindung an einen in die Hefezelle transfizierten Rezeptor kolorimetrisch gemessen wird.

Von Montmayeur und Borelli, 1991, wurde ein Assay beschrieben, der auf der Beeinflussung des Adenylatzyklase-Signalübertragungsweges durch Aktivierung von G-Protein-gekoppelten Rezeptoren (es wurden D₂-Rezeptoren und der β-adrenergische Rezeptor verwendet) beruht.

Himmler et al., 1993, haben einen funktionellen Test entwickelt, in dem die Aktivität der Rezeptor-Kopplung (humaner Dopamin D1- und D5- Rezeptor) an den cAMP-Signalübertragungsweg über die transkriptionelle Aktivierung eines Reportergens gemessen wurde.

Ausgehend von diesen Systemen lag der vorliegenden Erfindung die Aufgabe zugrunde, ein Screening-Verfahren bereitzustellen, mit dem Substanzen identifiziert werden können, die einen IL-5-Rezeptor-abhängigen Signalübertragungsweg, über dessen Mechanismen und Botenstoffe nur sehr wenig bekannt ist, modulieren. Insbesondere sollte mit Hilfe der vorliegenden Erfindung ein Verfahren bereitgestellt werden, das automatisierbar und somit zum Screenen von Substanzen in hohen Durchsatzraten geeignet ist und das auch die Untersuchung von komplexen Substanzgemischen, wie Extrakten von Organismen, auf ihren Gehalt an pharmakologisch wirksamen Substanzen erlaubt.

Die Identifikation von Substanzen aufgrund ihrer Eigenschaft, einen IL-5-Rezeptor-abhängigen Signalübertragungsweg zu modulieren, insbesondere von Antagonisten des IL-5-Rezeptors, bildet den Ausgangspunkt für die Entwicklung von Arzneimitteln, die für die Therapie von Erkrankungen eingesetzt werden, an denen die Modulation, häufig die Aktivierung, des IL-5-Rezeptors beteiligt ist.

Es konnte im Rahmen der vorliegenden Erfindung gezeigt werden, daß in Zellen, welche die für die Funktion des IL-5-Rezeptors erforderlichen Strukturelemente sowie die Komponenten des IL-5-Rezeptor-abhängigen Signalübertragungsweges exprimieren (derartige Zellen werden im folgenden der Einfachheit halber als "Zellen, die den funktionellen Il-5-Rezeptor exprimieren" bezeichnet) und die mit einem regulatorische DNA-Elemente enthaltenden Reportergenkonstrukt transformiert sind, das Reportergenkonstrukt durch Aktivierung des IL-5-Rezeptors nach Bindung seines Liganden induziert wird.

Dieses Ergebnis war aufgrund der geringen Kenntnisse über den IL-5-Rezeptor-abhängigen Signalübertragungsweg überraschend, zumal insbesondere auch keine Vorhersagen darüber möglich waren, ob bzw. welches der bisher charakterisierten regulatorischen DNA-Elemente auf einen Botenstoff des durch den IL-5-Rezeptor in Gang gesetzten Signalübertragunsweges ansprechen würde.

Die vorliegende Erfindung betrifft ein Verfahren zur Bestimmung der modulierenden Wirkung einer Substanz auf einen rezeptorabhängigen Signalübertragungsweg in der menschlichen oder tierischen Zelle, dadurch gekennzeichnet, daß man die modulierende Wirkung der Substanz auf eine Komponente im Signalübertragungsweg, der durch Aktivierung des Interleukin-5-Rezeptors ausgelöst wird, bestimmt, indem man Säugetierzellen, die
a) transformiert sind mit einer rekombinanten DNA, enthaltend ein Reportergen und eine regulatorische Sequenz, die auf eine Konzentrationsänderung eines oder mehrerer sekundärer Botenstoffe des Interleukin-5-Rezeptor-abhängigen Signalübertragungsweges anspricht, so daß die Expression des Reportergens durch eine Konzentrationsänderung des Botenstoffes moduliert wird, und die weiters
b) den funktionellen Interleukin-5-Rezeptor exprimieren, mit der zu untersuchenden Substanz inkubiert, die Konzentration des Reportergenproduktes mißt und untersucht, ob die festgestellte Wirkung der Testsubstanz selektiv für den Interleukin-5-Rezeptor-abhängigen Signalübertragungsweg ist, indem man die Substanz außerdem unter identischen Bedingungen auf Kontroll-Säugetierzellen aufbringt, die einen vom Interleukin-5-Rezeptor verschiedenen Kontroll-Rezeptor exprimieren und die mit einer rekombinanten DNA transformiert sind, die das Reportergen und eine regulatorische Sequenz enthält, die auf die Konzentrationsänderung eines sekundären Botenstoffes des Kontroll-Rezeptor-abhängigen Signalübertragungsweges anspricht, und daß man die Konzentration des Reportergenproduktes in den den Interleukin-5-Rezeptor exprimierenden Zellen mit der in den Kontrollzellen vergleicht.

Unter die Definition "Interleukin-5-Rezeptor-abhängiger Signalübertragungsweg" fällt im Rahmen der vorliegenden Erfindung auch ein Interleukin-5-Rezeptor-abhängiger Signalübertragungsmechanismus, der gegebenenfalls aus mehr als einem Signalübertragungsweg besteht.

Unter die Definition "Komponente im Signalübertragungsweg" fallen alle an Mechanismen des Signalübertragungsweges beteiligten Moleküle einschließlich des IL-5-Rezeptors selbst bzw. seiner Untereinheiten.

Wenn man nach Inhibitoren des IL-5-abhängigen Signalübertragungsweges sucht, werden die Testsubstanzen auf ihre antagonistische Wirkung untersucht, indem man sie zusammen mit IL-5 auf die Zellen bringt, wobei man im allgemeinen IL-5 gleichzeitig mit oder nach der Testsubstanz auf die Zellen aufbringt.

Die rekombinante DNA, welche auf Änderung der sekundären Botenstoffe anspricht und die ebenfalls Gegenstand der vorliegenden Erfindung ist, wird im folgenden als "Sensor-DNA" bezeichnet. Ein Reportergen ist dadurch definiert, daß sein Expressionsprodukt detektierbar und quantifizierbar durch Messung eines Signals ist, das seiner Konzentration proportional ist.

Zellen, die mit Sensor-DNA transformiert sind und den IL-5-Rezeptor exprimieren, werden im folgenden als "Testzellen" bezeichnet; diese Zellen sind ebenfalls Gegenstand der vorliegenden Erfindung.

Unter "IL-5-Rezeptor" wird im Rahmen der vorliegenden Erfindung der funktionelle IL-5-Rezeptor verstanden, wobei unter diese Definition auch gegebenenfalls vorhandene IL-5-Rezeptor-Subtypen fallen. Unter "funktioneller Rezeptor" ist im Rahmen der vorliegenden Erfindung einerseits der natürliche IL-5-Rezeptor, bestehend aus der α- und β-Untereinheit derselben Spezies, zu verstehen, ferner in den Testzellen, insbesondere Humanzellen, funktionsfähige Kombinationen von α- und β-Untereinheiten des IL-5-Rezeptors verschiedener Spezies, z.B. die α-Untereinheit des humanen und die β-Untereinheit des Maus-Rezeptors, oder umgekehrt. Ein funktioneller Rezeptor im Sinne dieser Definiton liegt ferner dann vor, wenn in einer Zelle, in der eine β-Untereinheit natürlicherweise mit der α-Untereinheit eines anderen Rezeptors, z.B. des IL-3- oder des GM-CSF-Rezeptors, interagiert, die α-Untereinheit des IL-5-Rezeptors in die Zelle eingeführt und zur Expression gebracht wird, wobei auch in diesem Fall die α-Untereinheit von einer anderen Spezies stammen kann, sofern im Hinblick auf die Auslösung des Signaltransduktionsweges das funktionelle Zusammenwirken der beiden Untereinheiten gewährleistet ist.

Die rekombinante DNA, die eine oder mehrere für die Untereinheit(en) des IL-5-Rezeptors kodierende Sequenz(en) enthält, wird im folgenden als "Rezeptor-DNA" bezeichnet.

Als Ausgangszellen für die Herstellung von Testzellen eignen sich Zellen, die den vollständigen funktionellen IL-5-Rezeptor bereits endogen enthalten, andererseits aber auch Zellen, die die für die Induktion der Sensor-DNA notwendigen Komponenten der an den Rezeptor gekoppelten Signaltransduktionswege enthalten. Für die Eignung solcher Zellen ist es nicht erforderlich, daß sie von vornherein den vollständigen IL-5-Rezeptor oder auch nur eine seiner Untereinheiten exprimieren. Bei Fehlen einer oder beider Untereinheiten kann die Zelle außer mit der Sensor-DNA zusätzlich mit der für die fehlende(n) Rezeptor-Untereinheit(en) kodierenden Sequenz transformiert werden.

In einer Ausführungsform der vorliegenden Erfindung ist die Testzelle von einer Zelle abgeleitet, die natürlicherweise beide für den funktionellen IL-5-Rezeptor erforderlichen Untereinheiten exprimiert.

In einer weiteren Ausführungsform der vorliegenden Erfindung ist die Testzelle von einer Ausgangszelle abgeleitet, die nur die β-Untereinheit exprimiert, in diesem Fall wird die Zelle außer mit Sensor-DNA mit einem Plasmid transformiert, das eine für die α-Untereinheit des IL-5-Rezeptors kodierende DNA-Sequenz aufweist.

In einer weiteren Ausführungsform der Erfindung enthält die Ausgangszelle für die Testzelle natürlicherweise weder die α- noch die β-Untereinheit des IL-5-Rezeptors. In diesem Fall muß die Zelle mit den beiden DNA-Sequenzen transformiert werden, die gemeinsam auf einem oder auch auf getrennten Plasmiden vorliegen können.

Wenn Substanzen im Hinblick auf ihre pharmakologische Wirkung für die Behandlung pathologischer Zustände des Menschen untersucht werden sollen, enthält die Rezeptor-DNA vorzugsweise die für den humanen IL-5-Rezeptor kodierende Sequenz. (Das erfindungsgemäße Verfahren dient bevorzugt zum Auffinden von Substanzen, die für die Behandlung von pathologischen Zuständen des Menschen geeignet sind. Es kann jedoch auch zum Screenen von Substanzen, die für die Behandlung von Tieren zum Einsatz kommen, verwendet werden; in diesem Fall wird der entsprechende tierische IL-5-Rezeptor verwendet.)

Vorzugsweise werden Zellen für die Herstellung von Testzellen danach ausgewählt, daß sie nach Stimulation mit Substanzen, die IL-5-Rezeptor-abhängig die Konzentration sekundärer Botenstoffe erhöhen, eine starke Expression des Reportergens zeigen. Dies wird zweckmäßigerweise dadurch geprüft, daß man Zellen, die einen funktionellen IL-5-Rezeptor exprimieren, mit Sensor-DNA transient transformiert und die Konzentration des Reporterproduktes nach Stimulation des Rezeptors mit IL-5 untersucht.

Als Ausgangszellen für Testzellen kommen insbesondere Zellen in Betracht, von denen bekannt ist, daß sie den IL-5-Rezeptor oder einen Rezeptor, der die β-Untereinheit enthält, exprimieren, bzw. Zellen, von denen aufgrund ihrer Herkunft bzw. Funktion im Organismus angenommen werden kann, daß sie einen der fraglichen Rezeptoren exprimieren, insbesondere hämatopoetische Vorläuferzellen. Die Expression des Rezeptors kann gegebenenfalls durch Bindungsassays und/oder durch Proliferationsassays, in denen das Wachstum der Zellen in Gegenwart der jeweiligen Liganden (IL-5, IL-3 oder GM-CSF) bestimmt wird, bestätigt werden.

Beispiele für geeignete Ausgangszellen sind aus Knochenmarkzellen etablierte faktor-abhängige Zellinien; die Herstellung derartiger Zellinien, die die β-Untereinheit exprimieren, wurde z.B. von Dexter et al., 1980, für Mauszellen beschrieben. Im Rahmen der vorliegenden Erfindung wurde ein Vertreter einer solchen Mauszellinie mit der α-Untereinheit des Maus-IL-5-Rezeptors transfiziert und als Testzelle verwendet. Ein weiteres Beispiel für geeignete Testzellen sind IL-5-abhängige hämatopoetischen Zellen, z.B. Zellen der von Kitamura et al., 1989 und 1991 beschriebenen Zellinie TF-1.

In Zellen, in denen beide Untereinheiten murinen Ursprungs sind, z.B. der Mauszellinie 1H3, kann die α- und gegebenenfalls auch die β-Untereinheit, z.B. durch homologe Rekombination, ausgeschaltet und durch die entsprechende Humansequenz ersetzt werden.

Als Ausgangszellen kommen grundsätzlich Säugetierzellen beliebiger Spezies in Betracht, wobei bevorzugt humane Zellen eingesetzt werden. Bei Verwendung nichthumaner Zellen werden bevorzugt solche eingesetzt, die mit humaner IL-5-Rezeptor-DNA (β- und/oder α-Untereinheit) transformiert sind.

Eine weitere Voraussetzung für die Eignung einer Zelle als Testzelle im Rahmen der vorliegenden Erfindung ist ihre Stabilität. Um die Stabilität der Zellen (Lebensfähigkeit, stabile Integration der Fremd-DNA ins Genom) zu testen, werden über einen längeren Zeitraum unter identischen Bedingungen mit den Testzellen Versuche durchgeführt und die Reproduzierbarkeit der Meßergebnisse überprüft.

Die Sensor-DNA befindet sich vorzugsweise auf einem Plasmid, das sich in einem geeigneten Wirtsorganismus, vorzugsweise E. coli, in hoher Kopienzahl vermehren läßt und nach Transfektion in Säugetierzellen und Integration ins Wirtsgenom die Expression eines Reportergens unter Kontrolle von regulatorischen Elementen ermöglicht. Es handelt sich dabei bevorzugt um einen Shuttle-Vektor, der eine Expressionskassette für das Reportergen (Sensor DNA) und einen selektionierbaren Marker für Säugetierzellen sowie mindestens einen Replikationsursprung und einen Marker für die Vermehrung und Selektion in E.coli enthält.

Zur Herstellung von permanenten Zellinien, die die Sensor-DNA stabil in ihr Genom integriert enthalten, enthält der Vektor einen dominanten Selektionsmarker. Die Verwendung eines bestimmten Selektionsmarkers ist nicht kritisch, geeignet sind z.B. das Gen für Neomycin-Phosphotransferase (neo), das eine Resistenz gegenüber dem Antibiotikum Geneticin (G-418) verleiht (Southern und Berg, 1982), das DHFR-Gen (Dihydrofolatreduktase) für DHFR-defiziente Zellen, das Gen für Xanthin-Guanin-Phosphoribosyltransferase (gpt), das Resistenz gegen Mycophenolsäure verleiht (Mulligan und Berg, 1981) oder das Hygromycin-B-Phosphotransferase-Gen (hph; Gritz und Davies, 1983). Beispiele für Promotoren, die das Selektionsmarker-Gen treiben, sind der SV40-Early-Promotor, der Zytomegalovirus-Promotor (CMV-Promotor), der Promotor des Thymidin-Kinase-Gens des Herpes simplex-Virus (TK-Promotor), der Rous Sarcoma Virus (RSV) long terminal repeat (LTR). Die Plasmide werden vorzugsweise derart konstruiert, daß einzelne wichtige Elemente, wie das Reportergen, der Promotor für das Reportergen, die regulatorischen Sequenzen für den Selektionsmarker, einfach ausgetauscht oder verändert werden können, um etwaigen geänderten Anforderungen, die sich aus dem speziellen Anwendungsfall ergeben, z.B. aufgrund der Verwendung einer anderen Zellinie, entsprechen zu können. Derartige Maßnahmen bestehen z.B. darin, vor den/die Promotor(en) oder vor das Reportergen Multiklonierstellen einzubauen, um die Klonierung von regulatorischen, den Promotor modulierenden Sequenzen bzw. von verschiedenen Reportergenen zu ermöglichen.

Bei der Auswahl eines geeigneten Reportergens wurde davon ausgegangen, einen, vorzugsweise nicht-radioaktiven, automatisierbaren Assay mit hoher Empfindlichkeit bereitzustellen.

Im Rahmen der vorliegenden Erfindung können grundsätzlich sämtliche Reportergene eingesetzt werden, die diese Voraussetzungen erfüllen:

Alkalische Phosphatase kann bei Verwendung eines chemilumineszierenden Substrats mit hoher Empfindlichkeit gemessen werden, weist allerdings den Nachteil auf, daß viele Säugetierzellen dieses Enzym relativ stark exprimieren. Es kommt daher als Reportergen im allgemeinen nur für solche Zellinien in Betracht, die es nicht oder nur schwach exprimieren.

Die Expressionsprodukte des β-Galaktosidase- und des β-Glucuronidase-Gens können das entsprechende Methylumbeliferyl-Galaktosid bzw. -Glucuronid unter Bildung fluoreszierender Gruppen spalten. Diese Enzymreaktionen werden mit Hilfe etablierter Fluoreszenz-Assays verfolgt (Wieland et al., 1985; Kricka, 1988).

Die Expression von Chloramphenicol-Actetyltransferase (CAT) ist zwar relativ empfindlich nachweisbar, der Assay weist jedoch u.a. den Nachteil auf, daß er radioaktiv und schwer automatisierbar ist (Hartmann, 1991).

Bevorzugt wird im Rahmen der vorliegenden Erfindung als Reportergen das für Photinus pyralis-Luciferase kodierende Gen (De Wet et al., 1987) verwendet. Dieses Enzym weist die Vorteile auf, daß es mit seinem Substrat Luciferin unter Zugabe von ATP Bioluminiszenz in hoher Ausbeute erzeugt, die mit Hilfe etablierter, automatisierbarer Methoden gemessen werden kann, und daß dieses Enzym von Säugetierzellen nicht endogen produziert wird. Weiters hat Luciferase eine relativ kurze in *vivo* Halbwertszeit und ist auch in hohen Konzentrationen nicht toxisch (Hartmann, 1991; Brasier et al., 1989).

Bei der Konstruktion der Sensor-DNA wird das Reportergen unter die Kontrolle von Promotoren gestellt, die modulierbare Regulationselemente, wie TRE- und/oder CRE- und/oder SRE- und/oder GRE-Elemente, enthalten. Da die Signaltransduktionswege des IL-5-Rezeptors im einzelnen nicht genau bekannt sind, werden die am besten geeigneten Sensor-DNA-Konstruktionen in Vorversuchen empirisch bestimmt. Dazu wird eine Zelle, die einen funktionellen IL-5-Rezeptor exprimiert, transient mit verschiedenen Sensor-DNA-Konstrukten transformiert, wobei einerseits hinsichtlich des Reportergens, andererseits hinsichtlich der Kontrollsequenzen variiert werden kann, und die Messung des Reportergen-Produktes auf seine Empfindlichkeit untersucht, indem die Konzentration des Reportergen-Produktes nach Stimulation des Rezeptors mit einem Agonisten, vorzugsweise mit IL-5, gemessen wird. Als regulatorische Sequenzen in der Sensor-DNA eignen sich sämtliche DNA-Elemente, durch die die Aktivierung des Promotors herbeigeführt wird, z.B. Elemente, ausgewählt aus der Gruppe TRE, CRE, GRE oder SRE oder auch solche, die eine Kombination dieser verschiedenen Elemente enthalten. Mit einer solchen, zwei oder mehrere verschiedene Elemente enthaltenden Sensor-DNA wird die Aktivierung mehrerer Signaltransduktionswege einzeln oder auch die parallele Aktivierung mehrerer Signaltransduktionswege erfaßt. Die Auswahl der Sensor-DNA für die Testzell-Entwicklung wird danach getroffen, daß eine möglichst hohe Induzierbarkeit des Reportergens in dem gegebenen Zellsystem erfolgt. Die regulatorische DNA-Region kann eine natürlich vorkommende Promotorregion sein, sie kann aber auch künstlich hergestellt werden.

Gegebenenfalls wird der komplette Promotor eines durch mehrere sekundäre Botenstoffe induzierbaren Gens verwendet, z.B. die 5'-regulatorische Sequenz des ICAM-1-Gens (Interzelluläres Adhäsionsmolekül 1) oder des c-fos-Gens. Ein natürlicher, auf Aktivierung des IL-5-Rezeptors ansprechender Promotor, der multiple regulatorische Elemente enthält, z.B. der ICAM-1-Promotor, der u.a. TRE-, NKKB-, SP1- und AP2-Elemente enthält (Voraberger et al.,) oder der c-fos-Promotor, der neben CRE- und SRE-Elementen u.a. als "SIF-E-Element" und als "AP₁-ähnliches Element" bezeichnete regulatorische Sequenzen enthält (Hipskind und Nordheim, 1991), kann daraufhin untersucht werden, welche seiner Elemente für die Aktivierung verantwortlich sind. Dazu kann so vorgegangen werden, daß künstliche Promotorsequenzen, die gegenüber dem natürlichen Promotor verschiedene Mutationen und/oder Deletionen aufweisen, auf Aktivierbarkeit untersucht werden. Ausgehend von den Ergebnissen solcher Untersuchungen kann der Promotor gewünschtenfalls weiter optimiert werden, indem z.B. das bzw. die als maßgeblich identifizierten Elemente in Mehrfachkopie verwendet werden.

Gegebenenfalls werden bekannte natürliche oder synthetische Promotoren modifiziert, indem sie auf die für die Promotorfunktion erforderliche Minimalsequenz gekürzt werden.

Bei der Konstruktion einer Sensor-DNA, in der das Reportergen von einem nicht-natürlichen Promotor getrieben wird, werden zweckmäßig schwachen Promotor-Elementen ein oder mehrere Elemente, z.b. TRE- und/oder CRE- und/oder SRE- und/oder GRE-Regulationselemente, vorgeschaltet, die den Promotor modulieren. Dem Fachmann sind die für die Expression in bestimmten Säugetierzellen geeigneten Kontrollsequenzen bekannt; die Auswahl kann zunächst aufgrund der einschlägigen Literatur getroffen werden (z.B. Landschulz et al., 1988, Turner und Tjian, 1989), eine Einengung bzw. Optimierung kann mit den oben angeführten, einfach durchzuführenden transienten Transfektionsexperimenten durchgeführt werden. Beispiele für geeignete Promotoren sind der β-Globin-Promotor und der TK-Promotor.

Bei der Auswahl der in der Sensor DNA enthaltenen regulatorischen Sequenz (einschließlich seiner flankierenden Sequenzen) wird von literaturbekannten Elementen (z.B. für TRE- oder CRE- Elemente Montminy et al., 1990; Deutsch et al., 1988) ausgegangen, die in Vorversuchen auf ihre Eignung im Hinblick auf eine empfindlich nachweisbare Induzierbarkeit des Reportergens in einem gegebenen Zellsystem untersucht werden. Beispiele für geeignete TRE-Elemente, einschließlich deren flankierende Sequenzen, sind die Sequenzen von Somatostatin, "vasoactive intestinal peptide", Zytomegalovirus Enhancer, Rinder Leukämievirus-Long Terminal Repeat (BLV LTR) (CRE-Elemente) bzw. ICAM-1, Kollagenase, Parathyroidhormon (TRE-Elemente). Falls die in den natürlichen Sequenzen enthaltenen Motive keine perfekte Konsensussequenz aufweisen, können sie, falls eine solche erforderlich oder gewünscht ist, und gegebenenfalls auch die benachbarten Sequenzen, durch Austausch eines oder mehrerer Nukleotide verändert werden.

Die regulatorischen Elemente und die sie flankierenden Sequenzen können synthetisch hergestellt werden oder natürlichen Ursprungs sein.

Um den modulierenden Effekt eines sekundären Botenstoffes auf die Reportergenexpression zu verstärken, wird gegebenenfalls ein Konstrukt verwendet, das mehrere, homologe oder heterologe, regulatorische Sequenzen in Tandem enthält. Bei der Anordnung der Einzelelemente des Konstrukts wird der Abstand der Elemente zueinander so gewählt, daß die Bindung des Transkriptionsfaktors an die Elemente gewährleistet ist. Der optimale Abstand der regulatorischen Elemente zueinander, bei dessen Bestimmung auch Überlegungen bezüglich der sterischen Anordnung zum Tragen kommen, wird in Vorversuchen empirisch ermittelt, gegebenenfalls auch der Abstand zu anderen regulatorischen-DNA-Elementen, die einen Einfluß auf die Transkription haben, z.B. von der TATA-Box. Bei multiplen Regulationssequenzen können die Elemente und/oder die flankierenden Sequenzen identisch oder, zumindest teilweise, verschieden sein, letztere Ausführungsform wird für die Tandem-Konstruktion bevorzugt.

Als das regulatorische Element flankierende Sequenzen, von denen festgestellt wurde, daß sie ebenfalls Einfluß auf die Regulationseigenschaften der Elemente haben, werden bevorzugt, insbesondere in deren unmittelbarer Umgebung, die das spezielle regulatorische Element natürlicherweise umgebenden Sequenzen verwendet (Montminy et al., 1990; Deutsch et al., 1988). Die Sequenz bzw. deren Anordnung wird empirisch ermittelt.

Gegebenenfalls befinden sich die Elemente der Sensor-DNA und das zur Selektion benutzte Markergen auf zwei getrennten Plasmiden, wovon eines das Reportergenkonstrukt (einschließlich der Expressionskontrollsequenz, die die regulatorische Sequenz enthält) und eines das Selektionsmarkergenkonstrukt enthält. (Beispiele für geeignete Selektionsmarkergenkonstrukte sind die Plasmide pRSVneo, pSV2neo, pRSVgpt, pSV2gpt, deren Aufbau einschlägigen Handbüchern, z.B. "Cloning Vectors", entnommen werden kann.) Im Fall der Verwendung von getrennten Plasmiden werden die Zellen mit den beiden Plasmiden co-transfiziert und auf den Marker selektioniert. Das Vorhandensein des Selektionsmarkers läßt den Schluß zu, daß die Zelle auch das Reportergenkonstrukt enthält, weil bekannt ist, daß Co-Transformation zweier Gene, die sich auf physikalisch nicht miteinander verbundenen DNA-Segmenten befinden, häufig zur Expression beider co-transformierter Gene führt (Winnacker, 1985).

Im Hinblick auf die im Testverfahren einzusetzende Meßanordnung ist es zweckmäßig, das Verhältnis zwischen maximaler Änderung und Normalwert des Meßsignals zu optimieren, vorzugsweise durch Veränderung der Konstruktion der Sensor-DNA, z.B. durch strukturelle Veränderung der Promotoranordnung. Das Background-Signal ist bevorzugt niedrig genug, um eine Induktion der Reportergenexpression mit hoher Empfindlichkeit zu erfassen, andererseits aber hoch genug, um die Nachweisgrenze im Hinblick auf die Negativkontrolle bestimmen zu können.

Für das Rezeptor-DNA-Konstrukt gelten grundsätzlich dieselben Überlegungen wie für das Sensor-DNA-Konstrukt mit dem Unterschied, daß die Rezeptor-Sequenz(en) bevorzugt unter die Kontrolle eines starken, konstitutiven Promotors gestellt werden. Die Rezeptor-DNA kann mit Hilfe konventioneller molekularbiologischer Methoden erhalten werden, z.B. indem man, ausgehend von einer RNA-Präparation aus Zellen, die den IL-5-Rezeptor exprimieren, mittels Reverse-Transkriptase-PCR die cDNA präpariert, wobei die Primer aufgrund der publizierten Sequenz(en) konstruiert wurden, und in einen geeigneten Vektor hineinkloniert. Die cDNA kann auch mittels PCR aus einer cDNA-Bibliothek herausamplifiziert werden.

Gegebenenfalls befinden sich auch im Falle der Rezeptor-DNA die für die Rezeptor-Untereinheit kodierende Sequenz und der dominante Selektionsmarker auf getrennten Plasmiden, mit denen die Zellen co-transformiert werden.

Die Transfektion der Zellen mit Sensor- bzw. Rezeptor-DNA wird mit üblichen Transfektionsmethoden vorgenommen (vgl. z.B. Potter et al. 1984; Felgner et al., 1987), bevorzugt werden die Elektroporations-, die Calciumphosphat-Präzipitations- oder die Lipofektionsmethode eingesetzt.

Zur Kontrolle der Selektivität einer Testsubstanz für den IL-5-Rezeptor werden zweckmäßigerweise für weitere Kontrollversuche Testzellen für verschiedene andere Rezeptoren hergestellt und mit der Substanz behandelt. Wenn eine Substanz nur den IL-5-Rezeptor spezifisch beeinflussen darf, was im Hinblick auf die Spezifität eines Arzneimittels im allgemeinen der Fall ist, darf die Substanz nur diesen einen Rezeptor modulieren.

In den Kontrollzellen kann der Kontrollrezeptor prinzipiell entweder endogen vorhanden sein oder andernfalls in die Zelle transformiert werden. Wird die für die IL-5-Rezeptor-Testzelle verwendete Sensor-DNA auch durch Stimulation dieses Rezeptors durch einen Agonisten, z.B. IL-5, meßbar induziert, ist als Kontrollzelle die IL-5-Rezeptor-Testzelle geeignet, wenn die für den Kontrollrezeptor kodierende Sequenz endogen vorhanden ist oder zusätzlich in die Zelle transformiert wurde. (Unter Kontrollrezeptoren sind auch solche Rezeptoren zu verstehen, die mit dem IL-5-Rezeptor die β-Untereinheit gemeinsam haben, z.B. der IL-3-Rezeptor.) Ebenso können in diesem Fall als Kontrollzellen Zellen verwendet werden, die diese Sensor-DNA und den Kontrollrezeptor, nicht jedoch den IL-5-Rezeptor enthalten. Wird die für die IL-5-Rezeptor-Testzelle verwendete Sensor-DNA dagegen nicht durch Stimulation des Kontrollrezeptors induziert, muß eine geeignete Sensor-DNA empirisch bestimmt werden, wobei von literaturbekannten Elementen (s. z.B. Montminy et al., 1990; Deutsch et al.,1988) ausgegangen wird, die in Vorversuchen auf ihre Eignung im Hinblick auf eine empfindlich nachweisbare Induzierbarkeit des Reportergens in dem gegebenen Zellsystem untersucht werden. Die Sensor-DNA wird dann, zusammen mit der kodierenden Sequenz für den Kontrollrezeptor, sofern dieser nicht endogen vorhanden ist, in die Zelle transformiert.

Die Selektivität der modulierenden Wirkung der Substanz auf den Interleukin-5-Rezeptor-abhängigen Signalübertragungsweg wird vorzugsweise überprüft, indem man sie unter identischen Bedingungen auf Kontrollzellen aufbringt, die einen Kontroll-Rezeptor exprimieren und die mit einer Sensor-DNA transformiert sind, deren regulatorische Sequenz nicht nur auf den Interleukin-5-Rezeptor, sondern auch auf die Aktivierung des Kontroll-Rezeptors anspricht, und daß man die Konzentration des Reportergenproduktes in den den Interleukin-5-Rezeptor exprimierenden Zellen mit der in den Kontrollzellen vergleicht.

Nach Transformation der Zellen mit Rezeptor-DNA werden die positiven Klone, z.B. mit Hilfe von Bindungsassays, in denen bekannte radioaktiv markierte Agonisten und Antagonisten eingesetzt werden, auf die Höhe der Expression des Rezeptors untersucht.

Mit Hilfe von Scatchard-Blots (Human Pharmacology, 1991) kann die Rezeptorzahl in Molekülen pro Zelle bestimmt werden.

Bevorzugt wird aus den stabilen, Rezeptor-DNA enthaltenden Transformanden ein Klon mit einer Rezeptorzahl gewählt, die der physiologischen Rezeptor-Konzentration möglichst entspricht. (Ist die Rezeptorzahl zu hoch, kann der Fall eintreten, daß unvollständige und möglicherweise unspezifische Kopplung stattfindet oder daß zusätzlich zur spezifischen Kopplung eine unspezifische Kopplung stattfindet, wodurch gegebenenfalls andere Effektorsysteme mitaktiviert werden. Ist die Rezeptorzahl zu niedrig, ist das Signal gegebenenfalls zu niedrig, um durch die Messung erfaßt zu werden.)

Unter "modulierender" Wirkung wird ein agonistischer oder antagonistischer Effekt auf einen IL-5-rezeptorabhängigen Signalübertragungsweg verstanden.

Substanzen, die im Testverfahren eine inhibierende Wirkung auf die IL-5-Rezeptor-abhängige Reportergen-Expression gezeigt haben, können in einem weiteren Verfahrensschritt darauf getestet werden, ob sie die IL-5-abhängige Proliferation von Zellen inhibieren. Bei einem solchen Verfahren bestimmt man die inhibierende Wirkung der Substanz auf die Proliferation der Zellen, indem man Zellen, die neben Rezeptoren für andere Wachstumsfaktoren den funktionellen IL-5- Rezeptor exprimieren und in ihrem Wachstum von der Anwesenheit von IL-5 mindestens des Liganden für einen dieser Rezeptoren, z.B. IL-5, abhängig sind, mit IL-5 allein sowie in einem weiteren Ansatz mit IL-5 und der zu testenden Substanz inkubiert und die Proliferation der beiden Ansätze mißt.

Methoden für solche Proliferationsassays stehen in großer Auswahl zur Verfügung, sie können u.a. in 96 Loch Mikrotiterplatten durchgeführt werden und sind auf einfache Weise zu automatisieren. Beispiele für derartige Assays sind solche beruhend auf der Messung des [³H]-Thymidin- oder Bromdesoxyuridin(BrdUrd)-Einbaus (Messung des BrdUrd-Einbaus z. B.über die Bindung von fluoreszenzmarkierten Anti-BrdUrd-Antikörper).
Ein weiteres Proliferationsassayprinzip beruht auf der Messung von Farbstoffen, die durch Umsetzung geeigneter Substrate durch Zellenzyme gebildet werden. Eine der auf diesem Prinzip entwickelten Methoden ist die kommerziell erhältliche Tetrazoliumsalz-Methode (MTT-Methode), bei der die Konzentration des durch Umsetzung eines Tetrazoliumsalzes durch Dehydrogenase gebildeten Farbstoffes Formazan spektrophotometrisch gemessen wird.

Beispiele für im Proliferationsassay geeignete Zellen sind IL-5-abhängige hämatopoetische Zellen, z.B. Zellen der von Kitamura et al, 1989 und 1991, beschriebenen Zellinie TF-1.

Bei den mit Hilfe des erfindungsgemäßen Verfahrens auf ihre potentielle pharmakologische Wirkung zu untersuchenden Substanzen handelt es sich um natürliche oder synthetische Substanzen, wobei Reinsubstanzen oder Substanzgemische (z.B. Pflanzenextrakte, Fermentationsbrühen, etc.) eingesetzt werden können. Bei den Reinsubstanzen sind insbesondere niedermolekulare synthetische organische Verbindungen von Interesse. Zu den Substanzen, die mit Hilfe eines Screenings erfaßt werden sollen, zählen prinzipiell Substanzen, die an die Liganden-Bindungsstelle des Rezeptors binden, allosterisch wirkende Substanzen sowie Substanzen, die in bezug auf die LigandenBindungsstelle nicht-kompetitiv wirken. Diese Substanzen können peptidischer und nicht-peptidischer Natur sein.

Zweckmäßig werden die Substanzen in seriellen Verdünnungen auf die Zellen aufgebracht, um einen möglichst großen Konzentrationsbereich zu erfassen. Die Inkubationszeit wird empirisch bestimmt, indem z.B. die gegebenen Testzellen mit bekannten Rezeptor-Agonisten behandelt werden und der Zeitpunkt bestimmt wird, ab dem die Induktion der Reportergenexpression reproduzierbar gemessen werden kann. Die Inkubationszeit wird im allgemeinen auf diesen Zeitpunkt abgestellt, sie beträgt im allgemeinen mindestens 1 h. Die Zellzahl richtet sich vor allem nach der Nachweisgrenze des Meßsignals und nach dem Wachstumsstadium, in dem sich die Zellen befinden, die Untergrenze wird außerdem durch die technisch bedingte Fähigkeit definiert, die Zellen gleichmäßig auf die Testeinheiten zu verteilen. Die Zellzahl beträgt im Fall der Verwendung von Mikrotiterplatten mit 96 Vertiefungen z.B. ca. 1000 bis ca. 100.000 Zellen pro Testeinheit, kann jedoch auch bei entsprechender Empfindlichkeit des Meßsignals und exakter Verteilbarkeit der Zellen geringer sein. Das Wachstumsstadium, in dem die Zellen eingesetzt werden, hängt von den zelltypspezifischen Eigenschaften der Ausgangszelle ab, weiters wird es vor allem durch den jeweiligen Rezeptor bestimmt (bei verschiedenen Rezeptoren kann dasselbe Effektorsystem je nach Wachstumsstadium unterschiedlich bzw. unterschiedlich stark aktiviert werden); Wachstumsstadium und Zellzahl werden somit ebenfalls in Vorversuchen empirisch ermittelt, indem die Kinetik der Reportergenexpression in Prätest- und Testzellen verschiedenen Wachstumsstadiums bestimmt wird.

Im Rahmen der vorliegenden Erfindung konnte gezeigt werden, daß Zellen, die den IL-5-Rezeptor exprimieren und die mit einer Sensor-DNA transformiert waren, auf den Zusatz von IL-5, dem natürlichen Liganden des IL-5-Rezeptors, ansprechen, indem die Expression des Reportergens induziert wird. (Es wurden einerseits murine Zellen, die die β-Untereinheit natürlicherweise exprimieren und die zusätzlich mit der α-Untereinheit transformiert waren, als auch humane Zellen, die beide für den funktionellen IL-5-Rezeptor notwendigen Einheiten natürlicherweise exprimieren, getestet.) Die verwendete Sensor-DNA enthielt als regulatorisches Element entweder die 1.3 kb 5'-regulatorische Region des humanen ICAM-1-Gens, die u.a. ein TRE-Element enthält, oder die 0.756 kb 5'-regulatorische Region des humanen c-fos-Gens, die u.a. ein TRE, ein CRE und ein SRE enthält. Wurden diese Zellen, die endogene IL-3 und GM-CSF Rezeptoren enthalten, mit IL-3 oder GM-CSF stimuliert, ließ sich ebenfalls eine Induktion der Reportergen-Expression messen. In Kontrollzellen, die bis auf das Fehlen der α-Untereinheit des IL-5-Rezeptors (die die Ligandenbindung an den Rezeptor vermittelt) identisch mit den vorher verwendeten waren, ließ sich die Expression des Reportergens ebenfalls durch Behandlung mit IL-3 oder GM-CSF, nicht jedoch mit IL-5 induzieren.

Mit Hilfe der vorliegenden Erfindung wird ein empfindliches und vielseitiges funktionelles Verfahren zum Auffinden von Substanzen bereitgestellt, die IL-5-Rezeptor-abhängig einen oder mehrere Signalübertragungswege in der Zelle spezifisch beeinflussen. Die mit Hilfe des erfindungsgemäßen Verfahrens ermittelten Substanzen dienen als Leitsubstanzen für die Entwicklung von Medikamenten für die Behandlung von Erkrankungen, die mit einer Fehlfunktion des IL-5-Rezeptors bzw. des daran gekoppelten Signalübertragungsweges assoziiert sind, und können in weiterer Folge in einem sekundären Screening, z.B. im Fall von Antagonisten in einem Proliferationsassay unter Verwendung von Zellinien, oder mit Primärzellen und erst im Anschluß daran im Tierversuch, näher auf ihre pharmakologischen Eigenschaften untersucht werden. Die Anzahl der benötigten Tiere wird somit durch den Einsatz des erfindungsgemäßen Verfahrens beträchtlich verringert.

Das erfindungsgemäße Verfahren bietet außerdem den Vorteil, daß es automatisierbar ist, indem die Beschickung der Zellkulturgefäße, z.B. Mikrotiterplatten mit 96 Vertiefungen, die Beschickung mit den Testsubstanzlösungen, die Inkubations- und Waschschritte sowie die Messung, z.B. im Fall von Luciferase als Reportergenprodukt mit einem Luminometer, mit Robotern durchgeführt werden. Das erfindungsgemäße Verfahren eignet sich somit für Screening-Programme mit hoher Durchsatz-Kapazität, wobei beispielsweise ca. 2000 Substanzen bzw. Substanzgemische pro Woche getestet werden können.

Mit Hilfe des erfindungsgemäßen Verfahrens ist es möglich, allosterisch wirkende Substanzen und bezüglich der Ligandenbindungsstelle nicht-kompetitiv wirkende Substanzen erfassen zu könnnen.

Mit Hilfe des erfindungsgemäßen Verfahrens ist es weiters möglich, Rezeptoren zu klonieren, die pharmakologisch oder biochemisch charakterisiert und für die Liganden bekannt sind. Dabei wird von cDNA-oder genomischen Banken ausgegangen, von denen Pools in die entsprechende Zellinie transformiert werden. Die Expression des Rezeptors wird durch eine Expression des Reportergens angezeigt, nachdem der Rezeptor durch Bindung eines Liganden aktiviert wurde.

### Figurenübersicht

- Fig. 1:: Sensor-DNA-Plasmid pBHluc1.3, enthaltend die Promotorregion des ICAM-1 Gens
- Fig. 2:: Sensor-DNA-Plasmid pBHfosluci, enthaltend die Promotorregion des c-fos-Gens
- Fig. 3:: Induktion von pBHluc1.3 und pBHfosluci durch Rezeptorstimulation in 1H3-Zellen
- Fig. 4:: Induktion von pBHluc1.3 durch Rezeptorstimulation in murinen FDC-Pl-Zellen
- Fig. 5:: Induktion von pBHluc1.3 durch Rezeptorstimulation in humanen TF-1-Zellen
- Fig. 6:: Induktion der Proliferation von TF-1-Zellen durch Rezeptor-Stimulation

### Beispiel 1

### Herstellung von Reporterplasmiden mit durch sekundäre Botenstoffe regulierbaren Elementen (Sensor-DNA)

### a) Konstruktion des Plasmids pBHluc1.3

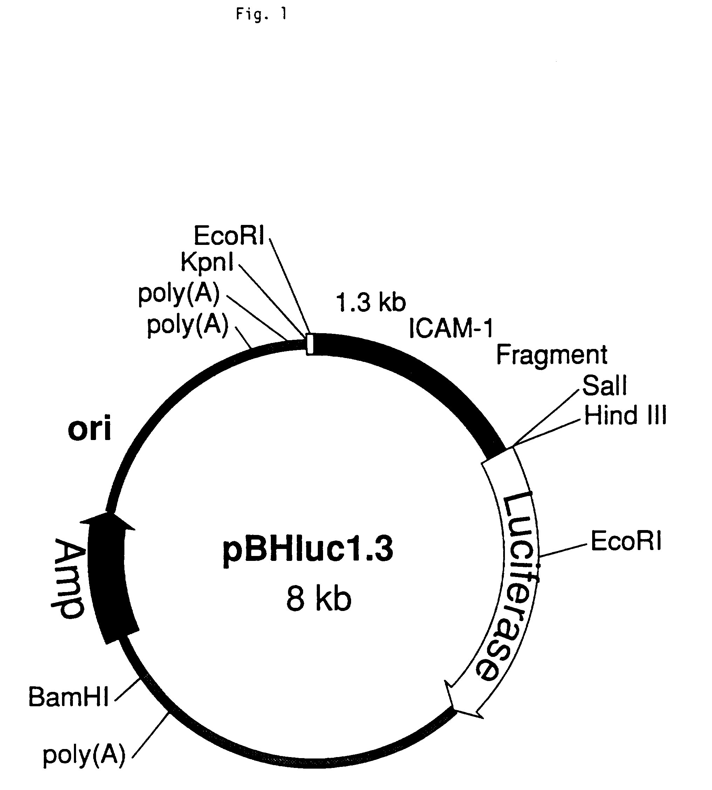

Klonierung und Deletionsanalyse von 1.3 kb der 5'-flankierenden Region des humanen Gens für das Interzelluläre Adhäsionsmolekül ICAM-1 haben ergeben, daß dieses Fragment i) in der Lungenadenokarzinom-Zellinie A549 (ATCC CCL 185) durch TPA induziert werden kann und ii) ein TPA Response Element (TRE) mit der DNA Sequenz TGATTCA enthält (Voraberger et al., 1991). Das Plasmid pBHlucl.3 (Fig.1 enthält die 1.3 kb lange Regulationsregion des ICAM-1 Gens, die dem Luciferase-Gen vorgeschaltet ist. Seine Herstellung wurde von Voraberger et al., 1991, beschrieben.

### b) Konstruktion des Plasmids pBHfosluci

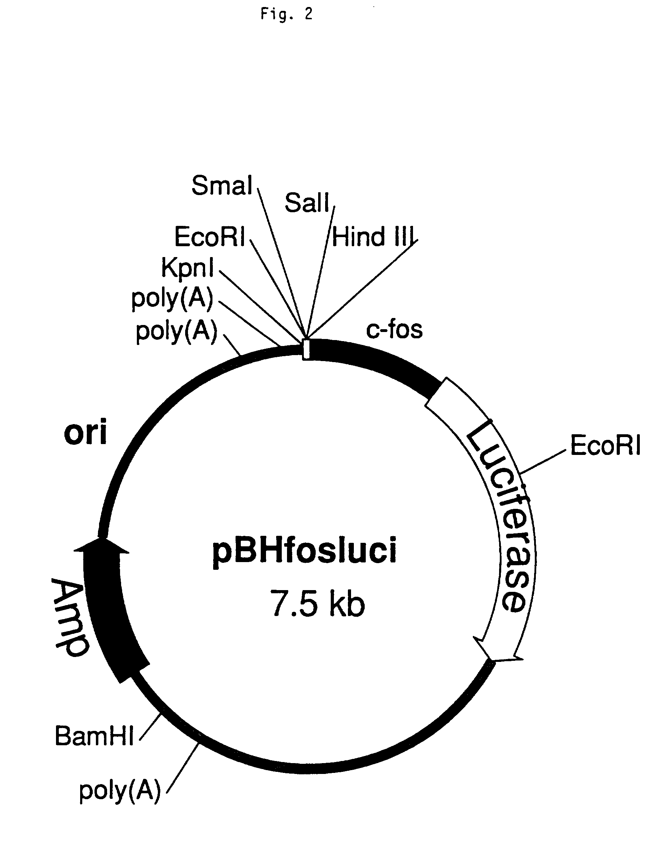

Das Plasmid pBHfosluci enthält das Luciferase-Gen unter der Kontrolle des Promotors des humanen c-fos Gens, der verschiedene Regulationselemente, z.B. TRE, CRE und ein SRE, enthält. Ein Fragment, das den c-fos Promotor (Position -711 bis +45) umfaßt, wurde aus dem Plasmid pfosCAT (Schönthal et al., 1988) isoliert, in dem Vektor pBluescriptSK (Stratagene) zwischenkloniert und in das Konstrukt pBHluc (Voraberger et al., 1991) vor das Luciferase-Gen inseriert.
Das Plasmid pfosCAT (Schönthal et al., 1988) wurde mit *Xb*aI und *Hind*III geschnitten. Das 756 bp Fragment, enthaltend den c-fos Promotor, wurde isoliert und mit dem Vektor pBluescriptSK, geschnitten mit *Xb*aI und *Hind*III, ligiert. Ein nach Transformation von E.coli erhaltenes Plasmid wurde pBSfos genannt. Das Plasmid pBSfos wurde mit XbaI geschnitten, die DNA-Enden des Plasmids durch Zugabe von Klenow-Enzym und aller 4 dNTPs "blunt" gemacht und schließlich mit *Sal*I geschnitten. Das *Sal*II/blunt Fragment, enthaltend den c-fos Promotor, wurde isoliert. Das Plasmid pBHluc (Voraberger et al., 1991) wurde mit *Hind*III geschnitten, die DNA-Enden wurden "blunt" gemacht, und es wurde mit *Sal*I geschnitten. Der so geschnittene Vektor wurde mit dem SalI/blunt Fragment, das den c-fos Promotor enthielt, ligiert. Das nach E.coli Transformation erhaltene Plasmid wurde pBHfosluci genannt (Fig.2).

### Beispiel 2

### Rezeptor-vermittelte Induktion der Sensor-DNA

Um zu zeigen, daß in Zellen, die den IL-5-Rezeptor exprimieren, die Sensor-DNA nach Stimulation des Rezeptors mit einer agonistisch wirkenden Substanz induziert wird, wurden Zellen einer murinen und einer humanen Zellinie mit Sensor-DNA (pBHluc1.3 oder pBHfosluci) transient transfiziert:

Murine 1H3-Zellen wurden aus FDC-Pl-Zellen, einer murinen, aus Knochenmark-Zellen etablierten faktor-abhängigen Zellinie (Dexter et al., 1980) durch Transformation mit der α-Untereinheit des murinen IL-5-Rezeptors folgendermaßen hergestellt: eine cDNA, kodierend für die α-Untereinheit des murinen IL-5-Rezeptors (Takaki et al., 1990), wurde in einer Reverse-Transkriptase-PCR-Reaktion (Frohman et al., 1988) unter Verwendung von RNA, präpariert aus der Zellinie BCL1, Klon 5Blb (ATCC TIB 197), synthetisiert. Die dazu verwendeten Primer waren MF71 (SEQ ID NO:1) (5'TCGGCTACCATGGTGCCTGTG3') und MF73 (SEQ ID NO:2) (5'ATGGCAAAATGCCATCAAAACGTG3'); sie flankieren die kodierende Region für die Rezeptor-α-Untereinheit (Takaki et al., 1990). (Im Sequenzprotokoll sind die synthetischen Oligodesoxiribonukleotide als "cDNS" angegeben.) Das 1272 bp PCR-Produkt wurde in die *Sma*I-Schnittstelle des Plasmids pUC19 (Pharmacia) subkloniert. Die kodierende Sequenz wurde mit *Bam*HI und *Eco*RI herausgeschnitten und in das Expressionsplasmid pcDNAI (Invitrogen), geschnitten mit den gleichen Enzymen, inseriert. 25 µg des linearisierten Plasmids wurden in 4x107 FDC-Pl-Zellen durch Elektroporation (300V, 960pF, BioRad Gene Pulser) transfiziert. IL-5-Rezeptor-exprimierende Zellen wurden in RPMI-1640-Medium, angereichert mit murinem IL-5, selektioniert.

1H3-Zellen exprimieren daher, im Gegensatz zu FDC-Pl-Zellen, die nur die IL-5-Rezeptor β-Untereinheit exprimieren, einen funktionellen IL-5-Rezeptor (α- plus β-Untereinheit). 1H3-Zellen und FDC-Pl-Zellen haben endogene IL-3 und GM-CSF Rezeptoren. Die β-Untereinheit ist bei IL-5-, IL-3- und GM-CSF-Rezeptoren identisch.

Die humane Zellinie TF-1 wurde aus Knochenmarkszellen eines Patienten mit Erythroleukämie etabliert, wie von Kitamura et al., 1989, beschrieben. Die Zellen exprimieren funktionelle Rezeptoren für IL-5, IL-3 und GM-CSF, und ihre Proliferation ist abhängig von der Anwesenheit mindestens eines der drei hämatopoetischen Wachstumsfaktoren (Kitamura et al, 1989, 1991).

Einen Tag nach der Transfektion der 1H3- bzw. TF-1-Zellen mit Sensor-DNA wurden die Zellen für etwa 12 Stunden in faktorfreiem Medium gehalten und anschließend mit dem Rezeptor-spezifischen Liganden IL-5 behandelt. Als negative Kontrolle für die 1H3-Zellen wurde das gleiche Experiment mit murinen FDC-Pl-Zellen, die keinen funktionellen IL-5-Rezeptor exprimieren, durchgeführt. In weiteren Kontroll-Experimenten wurden 1H3-, FDC-P1- bzw. TF-1-Zellen mit IL-3 oder GM-CSF anstelle von IL-5 behandelt.

### a) Induktion von pBHlucl.3 und pBHfosluci durch Rezeptor-Stimulation in 1H3 Zellen

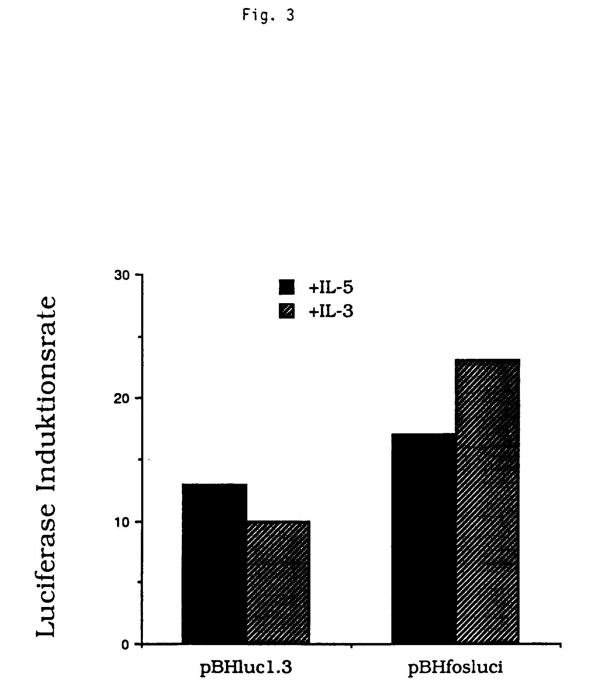

Die Zellinie 1H3, die einen funktionellen IL-5-Rezeptor exprimiert, wurde in RPMI 1640 Medium (Gibco) mit 10 % hitzeinaktiviertem fötalem Kälberserum (FCS) und 1 % Maus-Tonizitäts-Supplement (1M NaCl, 0.1M Natrium-Pyruvat, 11.3M Monothioglycerin) unter Zugabe von etwa 500 units/ml IL-5 (rekombinantes murines IL-5) bei 37°C in 5 % CO₂ gezüchtet. Etwa 3x10⁷ Zellen pro Transfektion wurden 5 min bei 1000 UpM abzentrifugiert und in 200 µl Medium mit allen Zusätzen resuspendiert. Nach Zugabe von 15 µg Plasmid-DNA pro 10⁷ Zellen wurde das Volumen auf 400 µl mit komplettem Medium aufgefüllt. Die Zellen wurden in einem Biorad Gene Pulser^{TM} mit einem Strompuls von 300V, 960µF durch Elektroporation transfiziert und anschließend in frischem, komplettem Medium mit IL-5 Zusatz über Nacht bei 37°C inkubiert. Am nächsten Tag wurden die Zellen 5 min bei 1000 UpM zentrifugiert und zweimal mit IL-5- freiem komplettem Medium gewaschen. Die Zellen wurden in IL-5- freiem komplettem Medium resuspendiert und über Nacht weiterinkubiert. Für die Induktion wurden die Zellen erneut zentrifugiert, in 60 ml faktorfreiem Medium resuspendiert und gleichmäßig auf drei Gewebekulturflaschen verteilt. Während die Zellen einer Flasche nicht induziert wurden und bis zum Zeitpunkt des Luciferase-Assays in faktorfreiem Medium gehalten wurden (negative Kontrolle), wurden die beiden anderen mit etwa 1000units/ml IL-5 bzw. IL-3 versetzt. Nach 8stündiger Inkubation bei 37°C wurden die Zellen abzentrifugiert, in PBS gewaschen und in 500 µl Lyse/Assay-Puffer (25 mM Tricine, 0.5 mM EDTA, 0.54 mM Natriumtripolyphosphat, 6.5 mM DTT, 16.3 mM MgSO₄.7H₂O, 0.1% Triton X-100, 1.2 mM ATP, 0.05 mM Luciferin; pH 7.8) aufgenommen. Davon wurden 400 µl unter Zugabe von 20 µl Luciferin (1mM) im Luminometer Lumat 9501 (Berthold) gemessen. Das Ergebnis des Experiments ist in Fig.3 zu sehen und zeigt, daß beide Sensor-Konstrukte, pBHluc1.3 und pBHfosluci, durch IL-5 mehr als 10fach induzierbar sind. Stimulation der Zellen mit IL-3 führt über endogene IL-3 Rezeptoren ebenfalls zu einer Induktion beider Sensor-Konstrukte.

### b) Induktion von pBHluc1.3 durch Rezeptor-Stimulation in FDC-P1 Zellen

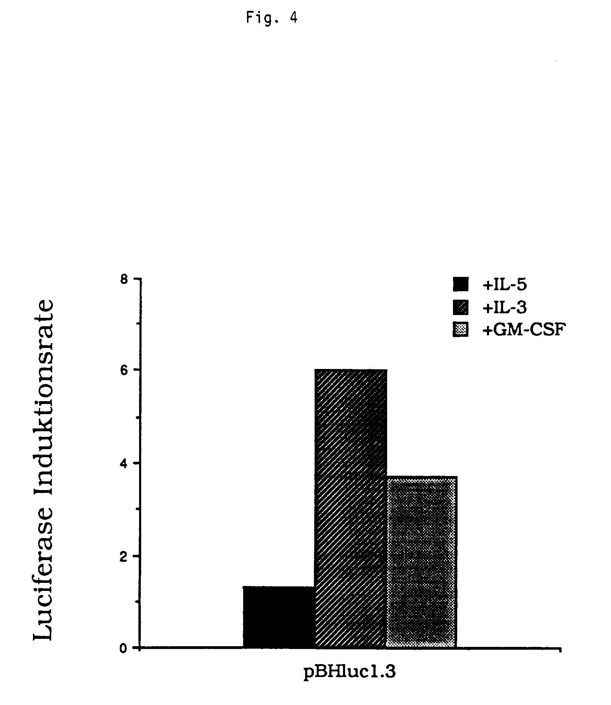

Die Zellinie FDC-P1 (Dexter et al., 1980) wurde in RPMI 1640 Medium (Gibco) mit den gleichen Zusätzen wie für 1H3 Zellen unter a) beschrieben gezüchtet, jedoch unter Zugabe von etwa 500units/ml IL-3 anstelle von IL-5. Etwa 4x10⁷ Zellen wurden wie unter a) beschrieben durch Elektroporation mit 15 µg pBHlucl.3 DNA pro 10⁷ Zellen transfiziert, inkubiert und in IL-3-freiem Medium gehalten. Die Zellen wurden auf vier Gewebekulturflaschen gleichmäßig verteilt. Die Zellen einer Flasche wurden bis zur Durchführung des Luciferase-Assays in faktorfreiem Medium gehalten (negative Kontrolle), die drei anderen wurden mit etwa 1000 Units/ml IL-5, IL-3 bzw. GM-CSF behandelt. Nach 8stündiger Inkubation wurde, wie unter a) beschrieben, der Luciferase-Assay durchgeführt. Das Ergebnis des Experiments ist in Fig.4 zu sehen. Die Sensor-DNA pBHluc1.3 ist in FDC-P1 Zellen, die nur die β-Untereinheit des IL-5-Rezeptors exprimieren, während die ligandenbindende α-Untereinheit fehlt, nicht durch IL-5 induzierbar. Im Gegensatz dazu führt IL-3 oder GM-CSF Behandlung der Zellen über Stimulation der wie in 1H3 Zellen vorhandenen endogenen IL-3 und GM-CSF Rezeptoren zu einer Induktion der Sensor-DNA.

### c) Induktion von pBHfosluci durch Rezeptor-Stimulation in TF-1-Zellen

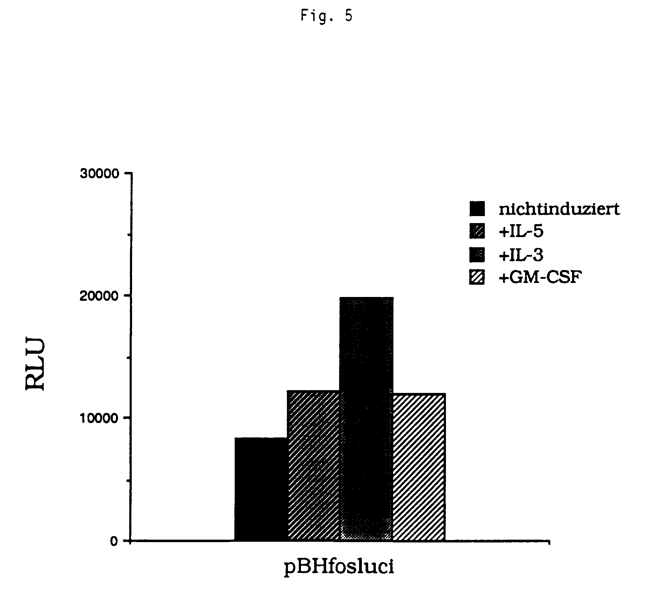

Die Zellinie TF-1 wurde in RPMI 1640 Medium (Gibco) mit 10 % hitzeinaktiviertem fötalem Kälberserum (FCS) unter Zugabe von 1 ng/ml IL-3 bei 37°C in 5 % CO₂ gezüchtet. Etwa 3 x 10⁷ Zellen pro Transfektion wurden 5 min bei 1000 UpM abzentrifugiert und in 200 µl Medium mit allen Zusätzen resuspendiert. Nach Zugabe von 15 µg/10⁷ Zellen Plasmid-DNA wurde das Volumen auf 400 µl mit komplettem Medium aufgefüllt. Die Zellen wurden in einem Biorad Gene Pulser^{TM} mit einem Strompuls von 280 V, 980 µF durch Elektroporation transfiziert und anschließend in frischem, komplettem Medium mit IL-3-Zusatz über Nacht bei 37°C inkubiert. Am nächsten Tag wurden die Zellen 5 min bei 1000 UpM zentrifugiert und zweimal mit IL-3-freiem komplettem Medium gewaschen. Die Zellen wurden in faktorfreiem komplettem Medium resuspendiert und über Nacht weiterinkubiert. Für die Induktion wurden die Zellen erneut zentrifugiert, in 60 ml faktorfreiem Medium resuspendiert und gleichmäßig auf vier Gewebekulturflaschen verteilt. Während die Zellen einer Flasche nicht induziert wurden und bis zum Zeitpunkt des Luciferase-Assays in faktorfreiem Medium gehalten wurden (negative Kontrolle), wurden die drei anderen mit etwa 10 ng/ml IL-5 bzw. 1 ng/ml IL-3 bzw. 100 ng/ml GM-CSF versetzt. Nach 8stündiger Inkubation bei 37°C wurden die Zellen abzentrifugiert, in PBS gewaschen und in 500 µl Lyse/Assay-Puffer (25 mM Tricin, 0.5 mM EDTA, 0.54 mM Natriumtripolyphosphat, 6.5 mM DTT, 16.3 mM MgSO₄.7 H₂O, 0.1% Triton X-100, 1.2 mM ATP, 0.05 mM Luciferin; pH 7.8) aufgenommen. Davon wurden 400 µl unter Zugabe von 20 µl Luciferin (1 mM) im Luminometer Lumat 9501 (Berthold) gemessen. Das Ergebnis des Experiments ist in Fig. 5 zu sehen und zeigt, daß das Sensor-Konstrukt pBHfosluci durch IL-5 sowie ebenfalls durch IL-3 und GM-CSF induzierbar ist.

### d) Induktion der Proliferation von TF-1-Zellen durch Rezeptor-Stimulation

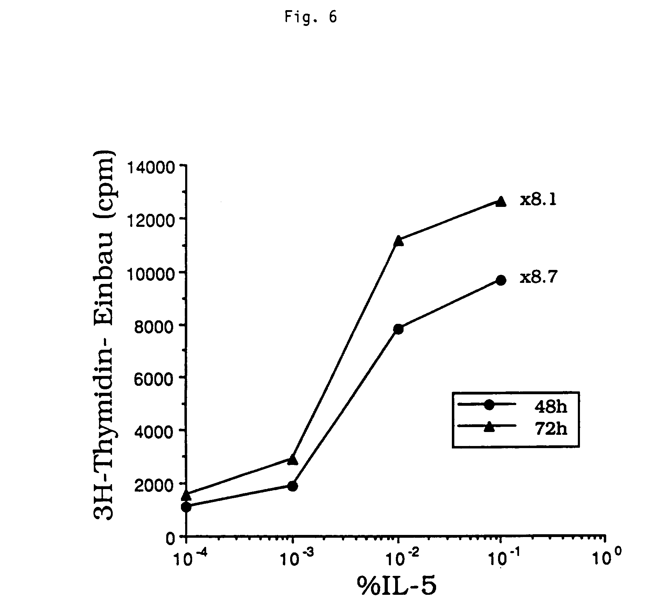

Um zu zeigen, daß TF-1-Zellen, die den IL-5-Rezeptor exprimieren, nach Stimulation des Rezeptors mit einer agonistisch wirkenden Substanz proliferieren, wurden die Zellen in 96well-Mikrotiterplatten ausgesät. Es wurden zwei Platten mit etwa 3 x 10³ Zellen in 100 µl RPMI 1640 Medium mit 2 % FCS pro Vertiefung angesetzt. Auf jeder Platte wurden drei Ansätze ohne IL-5 inkubiert (Negativkontrolle) und jeweils drei Ansätze mit 0.001 %, 0.01 % bzw. 0.1 % rekombinantem humanem IL-5 versetzt. Die Ansätze der ersten Platte wurden nach einer Inkubationszeit von 48 h, die der zweiten Platte nach 72 h Inkubation bei 37°C mit 1 µCi pro Vertiefung [6-³H]Thymidin (Amersham) versetzt und jeweils für weitere 12 h inkubiert. Anschließend wurden die Platten bei -20°C aufbewahrt bis zur Messung des Thymidin-Einbaus mittels eines Packard FilterMate Cell Harvesters und TopCount Microplate Szintillation Counters. Das Ergebnis ist in Fig. 6 zu sehen. Der Thymidin-Einbau erreicht bei der höchsten IL-5-Konzentration (0.1 %) nach 48stündiger Inkubationszeit das 8.lfache, nach 72 h das 8.7fache des Wertes der Kontrollzellen in IL-5-freiem Medium.

### Literatur:

Akira, S., Isshiki, H., Sugita, T., Tanabe, O., Kinoshita, S., Nishio, Y., Nakajima, T., Hirano, T. und Kishimoto, T., 1990, EMBO J. 9, 1897-1906.
Angel, P., Baumann, I., Stein, B., Delius, H., Rahmsdorf, H.J. und Herrlich, P., 1987a, Mol. Cell. Biol. 7, 2256-2266.
Angel, P., Imagawa, M., Chiu, R., Stein, B., Imbra, R.J., Rahmsdorf, H.J., Jonat, L., Herrlich, P. und Karin, M., 1987b, Cell 49, 729-739.
Billah, M.M., Pai, J.-K., Mullmann, T.J., Egan, R.W. und Siegel, 1989, J. Biol. Chem. 264, 9069-9076.
Brasier, A.R., Tate, J.E. und Habener, J.F., 1989, BioTechniques 7, 1116-1122.
Carroll, M.P., Clark-Lewin, I., Rapp, U.R. und May, W.S., 1990, J. Biol. Chem. 265, 19812-19817.
Deutsch, P.J., Hoeffler, J.P., Jameson, J.L. und Habener, J.F., 1988, Proc.Natl.Acad.Sci. USA 85, 7922-7926.
De Wet, J.R., Wood, K., DeLuca, M., Helinski, D. und Subramani, S., 1987, Mol. Cell. Biol. 7, 725-737.
Devos, R. et al., 1991, EMBO J. 10, 2133-2137.
Dexter , T.M., Garland, J., Scott, D., Scolnick, E. und Metcalf, D., 1980, J. Exp. Med. 152, 1036-1047.
Felgner, P.L., Gadek, T.R., Holm, M., Roman, R., Chan, H.N., Wenz, M., Northrop, J.P., Ringold, G.M., und Danielsen, M., 1987, Proc.Natl.Acad.Sci. USA 84, 7413-7417.
Frohman et al., 1988, Proc.Natl.Acad.Sci. USA 85, 8998-9002.
Gillis, S., 1991, Current Opinion in Immunology 3, 315-319.
Gritz, L. und Davies, J., 1983, Gene 25, 179-188.
Hanazono, Y., Chiba, S., Sasaki, K., Mano, H., Miyajima, A., Arai, K.-i., Yazaki, Y. und Hirai, H., 1993, EMBO J. 12, 1641-1646.
Hartmann, A., 1991, BioTec 5, 40-45.
Hatakeyama M., Mori, H., Doi, T. und Taniguchi, T., 1989, Cell 59, 837-845.
Hatakeyama M., Kawahara, A., Mori, H., Shibuya, H. und Taniguchi, T., 1992, Proc.Natl.Acad.Sci. USA 89, 2022-2026.
Himmler, A., et al., 1993, Journal of Receptor Research 13, 79-94.
Hipskind, R.A. und Nordheim, A., 1991, J. Biol. Chemistry 266, 19583-19592.
Houslay, M.D., 1991, Eur. J. Biochem. 195, 9-27.
Isfort, R., Huhn, R.D., Frackelton, A.R., Jr. und Ihle, J.N., 1988, J. Biol. Chem. 263, 19203-19209.
Jantzen et al., 1987, Cell 49, 29-38.
Julius, D., Huang, K.N., Livelli, T.J., Axel, R. und Jessell, T.M., 1990, Proc.Natl.Acad.Sci. USA 87, 928-932.
King, K., Dohlman, H.G., Thorner, J., Caron, M.G. und Lefkowitz, R.J., 1990, Science 250, 121-123.
Kitamura, T., Tange, T., Terasawa, T., Chiba, S., Kuwaki, T., Miyagawa, K., Piao, Y.-F., Miyazono, K., Urabe, A. und Takaku, F., 1989, J. Cell. Physiolog. 140, 323-334.
Kitamura, T., Takaku, F. und Miyajima, A., 1991, International Immunology 3, 571-577.
Koyasu, S., Tojo, A., Miyajima, A., Akiyama, T., Kasuga, M., Urabe, A., Schreurs, J., Arai, K-i., Takaku, F. und Yahara, I., 1987, EMBO J. 6, 3979-3984.
Kricka, L.J., 1988, Analyt. Biochem. 175, 14-21.
Lee, W., Mitchell, P. und Tjian, R., 1987, Cell 49, 741-752.
Landschulz, W.H., Johnson, P.F. und McKnight, S.L., 1988, Science 240, 1759-1764.
Milburn, M.V., Hassell, A.M., Lambert, M.H., Jordan, St.R., Proudfoot, A.E.I., Graber, P. und Wells T.N.C., 1993, Nature 363, 172-176.
Miyajima, A., Kitamura, T., Harada, N., Yokota, T. und Arai, K., 1992, Annu. Rev. Immunol. 10, 295-311.
Montmayeur, J.-P. und Borrelli, E., 1991, Proc.Natl.Acad.Sci. USA 88, 3135-3139.
Montminy, M.R., Gonzalez, G.A. und Yamamoto, K.K., 1990, Trends Neurosci. 13, 185.
Morla, A.O., Schreurs, J., Miyajima, A. und Wang, J.Y.J., 1988, Mol. Cell. Biol. 8, 2214-2218
Mulligan, R. und Berg, P., 1981, Proc.Natl.Acad.Sci. USA 78, 2072-2076.
Murata, Y., Yamaguchi, N., Hitoshi, Y., Tominaga, A. und Takatsu, K., 1990, Biochem. Biophys. Res. Commun. 173, 1102-1108.
Murata, Y., Takaki, S., Migita, M., Kikuchi, Y., Tominaga, A. und Takatsu, K., 1992, J. Exp. Med. 175, 341-351.
Potter, H., Weir, L., und Leder, P., 1984, Proc.Natl.Acad.Sci. USA 81, 7161.
Sassone-Corsi, P., Ransone, L.J. und Verma, I.M., 1990, Oncogene 5, 427-431.
Satoh, T., Nakafuku, M., Miyajima, A. und Kaziro, Y., 1991, Proc.Natl.Acad.Sci. USA 88, 3314-3318.
Satoh, T., Uehara, Y. und Kaziro, Y., 1992, J. Biol. Chem. 267, 2537-2541.
Scheidereit et al., 1986, DNA 5, 383-391.
Schönthal, A. et al., 1988, Cell 54, 325-334.
Southern, P. und Berg, P., 1982, J. Mol. Appl. Gen. 1, 327.
Strähle, U. et al., 1987, Proc.Natl.Acad.Sci. USA 84, 7871-7875.
Takaki, S., Tominaga, A., Hitoshi, Y., Mita, S., Sonoda, E., Yamaguchi, N. und Takatsu, K., 1990, EMBO J. 9, 4367-4374.
Takaki, S., Mita, S., Kitamura, T., Yonehara, S., Yamaguchi, N., Tominaga, A., Miyajima, A. und Takatsu, K., 1991, EMBO J. 10, 2833-2838.
Tavernier, J., Devos, R., Cornelis, S., Tuypens, T., Van der Heyden, J., Fiers, W. und Plaetinck, G., 1991, Cell 66, 1175-1184.
Tominaga, A., Takaki, S., Hitoshi, Y. und Takatsu, K., 1992, BioEssays 14, 527-533.
Treisman, R., 1985, Cell 42, 889-902.
Turner R. und Tjian, R., 1989, Science 243, 1689-1694.
Verma, I.M. und Sassone-Corsi, P., 1987, Cell 51, 513-514.
Voraberger, G., Schäfer, R. und Stratowa, Ch., 1991, J. Immunol. 147, 2777.
Welham, M.J. und Schrader, J.W., 1992, J. Immunol. 149, 2772-2783.
Whetton, A.D., Monk, P.N., Consalvey, S.D., Huang, S.J., Dexter, T.M. und Downes, C.P., 1988, Proc.Natl.Acad.Sci. USA 85, 3284-3288.
Wieland, E. et al., 1985, Ärztl. Lab. 31, 203-214.
Winnacker, E.L., 1985, Gene und Klone, Eine Einführung in die Gentechnologie, VCH Verlagsges. Weinheim, 264.
Yamamoto, K.R., 1985, Annu. Rev. Genet. 19, 209-252.
Cloning Vectors: Chapter VIII, Hsg. Pouwels, Enger-Valk, Brammer, Elsevier, Amsterdam, New York, Oxford, 1985.
Human Pharmacology - Molecular-To-Clinical, Chapter 2: Sites of Action: Receptors, Hsg. Wingard., L.B., Brody, T.M., Larner, J. und Schwartz, A., Mosby Year-Book Inc., St.Louis, 1991.

## Patentansprüche

1. Verfahren zur Bestimmung der modulierenden Wirkung einer Substanz auf einen rezeptorabhängigen Signalübertragungsweg in der menschlichen oder tierischen Zelle, dadurch gekennzeichnet, daß man die modulierende Wirkung der Substanz auf eine Komponente im Signalübertragungsweg, der durch Aktivierung des Interleukin-5-Rezeptors ausgelöst wird, bestimmt, indem man Säugetierzellen, die
a) transformiert sind mit einer rekombinanten DNA, enthaltend ein Reportergen und eine regulatorische Sequenz, die auf eine Konzentrationsänderung eines oder mehrerer sekundärer Botenstoffe des Interleukin-5-Rezeptor-abhängigen Signalübertragungsweges anspricht, so daß die Expression des Reportergens durch eine Konzentrationsänderung des Botenstoffes moduliert wird, und die weiters
b) den funktionellen Interleukin-5-Rezeptor exprimieren,
mit der zu untersuchenden Substanz inkubiert, die Konzentration des Reportergenproduktes mißt und untersucht, ob die festgestellte Wirkung der Testsubstanz selektiv für den Interleukin-5-Rezeptor-abhängigen Signalübertragungsweg ist, indem man die Substanz außerdem unter identischen Bedingungen auf Kontroll-Säugetierzellen aufbringt, die einen vom Interleukin-5-Rezeptor verschiedenen Kontroll-Rezeptor exprimieren und die mit einer rekombinanten DNA transformiert sind, die das Reportergen und eine regulatorische Sequenz enthält, die auf die Konzentrationsänderung eines sekundären Botenstoffes des Kontroll-Rezeptor-abhängigen Signalübertragungsweges anspricht, und daß man die Konzentration des Reportergenproduktes in den den Interleukin-5-Rezeptor exprimierenden Zellen mit der in den Kontrollzellen vergleicht.

2. Verfahren nach Anspruch 1, dadurch gekennzeichnet, daß man die Testsubstanz auf ihre antagonistische Wirkung für den Interleukin-5-Rezeptor-abhängigen Signalübertragungsweg untersucht, indem man außerdem Interleukin-5 auf die Zellen aufbringt.

3. Verfahren nach Anspruch 1, dadurch gekennzeichnet, daß man Zellen einsetzt, die den funktionellen Interleukin-5-Rezeptor natürlicherweise exprimieren.

4. Verfahren nach Anspruch 1, dadurch gekennzeichnet, daß man Zellen einsetzt, die natürlicherweise nur die β-Untereinheit des Interleukin-5-Rezeptors exprimieren und die mit einer rekombinanten DNA transformiert sind, die die für die α-Untereinheit des Interleukin-5-Rezeptors kodierende Sequenz enthält, so daß die Zellen den funktionellen Interleukin-5-Rezeptor exprimieren.

5. Verfahren nach Anspruch 1, dadurch gekennzeichnet, daß man Zellen einsetzt, die mit rekombinanter DNA transformiert sind, die die für die α-Untereinheit und die für die β-Untereinheit des Interleukin-5-Rezeptors kodierenden Sequenzen enthalten, so daß sie den funktionellen Interleukin-5-Rezeptor exprimieren.

6. Verfahren nach Anspruch 5, dadurch gekennzeichnet, daß die für die α-Untereinheit und die für die β-Untereinheit kodierenden DNA-Sequenzen auf zwei getrennten Plasmiden vorliegen.

7. Verfahren nach einem der Ansprüche 1 bis 6, dadurch gekennzeichnet, daß die Zellen einen funktionellen humanen Interleukin-5-Rezeptor exprimieren.

8. Verfahren nach einem der Ansprüche 1 bis 6, dadurch gekennzeichnet, daß die Zellen einen funktionellen murinen Interleukin-5-Rezeptor exprimieren.

9. Verfahren nach einem der Ansprüche 1 bis 6, dadurch gekennzeichnet, daß die Zellen einen funktionellen Interleukin-5-Rezeptor exprimieren, dessen α-Untereinheit und dessen β-Untereinheit von verschiedenen Spezies stammen.

10. Verfahren nach Anspruch 9, dadurch gekennzeichnet, daß die α-Untereinheit humanen Urspungs ist.

11. Verfahren nach einem der Ansprüche 1 bis 10, dadurch gekennzeichnet, daß die in a) definierte rekombinante DNA als regulatorische Sequenz den Promotor des c-fos-Gens enthält.

12. Verfahren nach einem der Ansprüche 1 bis 10, dadurch gekennzeichnet, daß die in a) definierte rekombinante DNA als regulatorische Sequenz den Promotor des ICAM-1-Gens enthält.

13. Verfahren nach Anspruch 11 oder 12, dadurch gekennzeichnet, daß die in a) definierte rekombinante DNA als Reportergen ein Luciferasegen enthält.

14. Verfahren nach einem der Ansprüche 2 bis 13, dadurch gekennzeichnet, daß man die Testsubstanz auf ihre antagonistische Wirkung untersucht, indem man außerdem Zellen, die neben Rezeptoren für andere Wachstumsfaktoren den funktionellen Interleukin-5-Rezeptor exprimieren und in ihrem Wachstum von der Anwesenheit mindestens des Liganden für einen dieser Rezeptoren abhängig sind, einerseits mit Interleukin-5 allein und andererseits mit Interleukin-5 zusammen mit der Testsubstanz inkubiert und die Proliferation der beiden Zellkulturen vergleicht.

15. Verfahren nach einem der Ansprüche 1 bis 13, dadurch gekennzeichnet, daß man die Selektivität der modulierenden Wirkung der Substanz auf den Interleukin-5-Rezeptor-abhängigen Signalübertragungsweg überprüft, indem man die Substanz außerdem unter identischen Bedingungen auf Kontroll-Säugetierzellen aufbringt, die einen vom Interleukin-5-Rezeptor verschiedenen Kontroll-Rezeptor exprimieren und die mit einer in a) definierten rekombinanten DNA transformiert sind, deren regulatorische Sequenz nicht nur auf eine Konzentrationsänderung eines oder mehrerer sekundärer Botenstoffe des Interleukin-5-Rezeptor-abhängigen Signalübertragungsweges, sondern auch des Kontroll-Rezeptor-abhängigen Signalübertragungsweges anspricht, und daß man die Konzentration des Reportergenproduktes in den den Interleukin-5-Rezeptor exprimierenden Zellen mit der in den Kontrollzellen vergleicht.

16. Verfahren nach Anspruch 15, dadurch gekennzeichnet, daß der Kontroll-Rezeptor der Interleukin-3-Rezeptor ist.

17. Verfahren nach einem der Ansprüche 1 bis 13, dadurch gekennzeichnet, daß man außerdem Säugetierzellen, die mit der in a) definierten rekombinanten DNA transformiert sind und keinen funktionellen Interleukin-5-Rezeptor exprimieren, unter identischen Bedingungen mit der zu untersuchenden Substanz inkubiert und die Konzentration des Reportergenproduktes mißt.

18. Verfahren nach einem der Ansprüche 1 bis 17, dadurch gekennzeichnet, daß es als Screening-Assay eingesetzt wird, in dem die zu untersuchende Substanz eine von zahlreichen Substanzen ist, mit denen man eine vorbestimmte Zahl von Säugetierzellen unter vorbestimmten Bedingungen inkubiert, die Konzentration des Reportergenproduktes mißt und die Selektivität der Substanzen auf den Interleukin-5 abhängigen Signalübertragungsweg überprüft.

19. Säugetierzellen, dadurch gekennzeichnet, daß sie
a) transformiert sind mit einer rekombinanten DNA, enthaltend ein Reportergen und eine regulatorische Sequenz, die auf eine Konzentrationsänderung eines oder mehrerer sekundärer Botenstoffe eines Interleukin-5-Rezeptor-abhängigen Signalübertragungsweges anspricht, und daß sie
b) transformiert sind mit rekombinanter DNA, kodierend für die α-Untereinheit oder für die α-und die β-Untereinheit des Interleukin-5-Rezeptors, so daß sie den funkionellen Interleukin-5-Rezeptor exprimieren.

20. Säugetierzellen nach Anspruch 19, dadurch gekennzeichnet, daß sie humane Zellen sind.

21. Säugetierzellen nach Anspruch 19, dadurch gekennzeichnet, daß sie murine Zellen sind.

22. Säugetierzellen nach einem der Ansprüche 19 bis 21, dadurch gekennzeichnet, daß sie natürlicherweise nur die β-Untereinheit des Interleukin-5-Rezeptors exprimieren und daß sie mit rekombinanter DNA transformiert sind, die die für die α-Untereinheit kodierende Sequenz enthält.

23. Säugetierzellen nach Anspruch 22, dadurch gekennzeichnet, daß die α-Untereinheit von einer anderen Spezies stammt.

24. Humane Zellen nach Anspruch 22, dadurch gekennzeichnet, daß sie mit rekombinanter DNA transformiert sind, die die für die humane α-Untereinheit kodierende Sequenz enthält.

25. Murine Zellen nach Anspruch 22, dadurch gekennzeichnet, daß sie mit rekombinanter DNA transformiert sind, die die für die murine α-Untereinheit kodierende Sequenz enthält.

26. Säugetierzellen nach einem der Ansprüche 19 bis 25, dadurch gekennzeichnet, daß die in a) definierte rekombinante DNA als regulatorische Sequenz den Promotor des c-fos-Gens enthält.

27. Säugetierzellen nach einem der Ansprüche 19 bis 25, dadurch gekennzeichnet, daß die in a) definierte rekombinante DNA als regulatorische Sequenz den Promotor des ICAM-1-Gens enthält.

28. Säugetierzellen nach einem der Ansprüche 19 bis 27, dadurch gekennzeichnet, daß die in a) definierte rekombinante DNA als Reportergen das Luciferasegen enthält.

## Claims

1. Process for determining the modulating effect of a substance on a receptor-mediated signal transduction pathway in a human or animal cell; characterised in that the modulating effect of the substance on a component in the signal transduction pathway which is triggered by activation of the interleukin-5 receptor is determined by incubating mammalian cells which are
a) transformed with a recombinant DNA, containing a reporter gene and a regulatory sequence which responds to a change in concentration of one or more secondary messenger substances of the interleukin-5 receptor mediated signal transduction pathway, so that the expression of the reporter gene is modulated by a change in concentration of the messenger substance, and which further
b) express the functional interleukin-5 receptor, with the substance which is to be investigated,
measuring the concentration of the reporter gene product and investigating whether the activity found for the test substance is selective for the interleukin-5 receptor mediated signal transduction pathway, by additionally applying the substance, under identical conditions, to mammalian control cells which express a control receptor other than the interleukin-5 receptor and which are transformed with a recombinant DNA which contains the reporter gene and a regulatory sequence which responds to the change in concentration of a secondary messenger substance of the control receptor mediated signal transduction pathway, and comparing the concentration of the reporter gene product in the cells which express the interleukin-5 receptor with that in the control cells.

2. Process according to claim 1, characterised in that the test substance is investigated for its antagonistic effect on the interleukin-5 receptor mediated signal transduction pathway, by also applying interleukin-5 to the cells.

3. Process according to claim 1, characterised in that cells are used which naturally express the functional interleukin-5 receptor.

4. Process according to claim 1, characterised in that cells are used which naturally express only the β-subunit of the interleukin-5 receptor and which are transformed with a recombinant DNA containing the sequence which codes for the α-subunit of the interleukin-5 receptor, so that the cells express the functional interleukin-5 receptor.

5. Process according to claim 1, characterised in that cells are used which are transformed with recombinant DNA, which contain the sequences coding for the α-subunit and those coding for the β-subunit of the interleukin-5 receptor, so that they express the functional interleukin-5 receptor.

6. Process according to claim 5, characterised in that the DNA sequences coding for the α-subunit and those coding for the β-subunit occur on two separate plasmids.

7. Process according to one of claims 1 to 6, characterised in that the cells express a functional human interleukin-5 receptor.

8. Process according to one of claims 1 to 6, characterised in that the cells express a functional murine interleukin-5 receptor.

9. Process according to one of claims 1 to 6, characterised in that the cells express a functional interleukin-5 receptor, the α-subunit and β-subunit of which originate from different species.

10. Process according to claim 9, characterised in that the α-subunit is of human origin.

11. Process according to one of claims 1 to 10, characterised in that the recombinant DNA defined in a) contains the promoter of the c-fos-gene as a regulatory sequence.

12. Process according to one of claims 1 to 10, characterised in that the recombinant DNA defined in a) contains the promoter of the ICAM-1-gene as a regulatory sequence.

13. Process according to claim 11 or 12, characterised in that the recombinant DNA defined in a) contains a luciferase gene as reporter gene.

14. Process according to one of claims 2 to 13, characterised in that the test substance is investigated for its antagonistic activity, by also incubating cells which express the functional interleukin-5 receptor as well as receptors for other growth factors and which are dependent for their growth on the presence of at least the ligand for one of these receptors, on the one hand with interleukin-5 on its own and on the other hand with interleukin-5 together with the test substance and comparing the proliferation of the two cell cultures.

15. Process according to one of claims 1 to 13, characterised in that the selectivity of the modulating effect of the substance on the interleukin-5 receptor mediated signal transduction pathway is tested by also applying the substance under identical conditions to mammalian control cells which express a control receptor other than the interleukin-5 receptor and which are transformed with a recombinant DNA defined in a), the regulatory sequence of which responds not only to any change in concentration of one or more secondary messenger substances of the interleukin-5 receptor mediated signal transduction pathway but also to any change in concentration of the control receptor-dependent signal transduction pathway, and comparing the concentration of the reporter gene product in the cells which express the interleukin-5 receptor with that in the control cells.

16. Process according to claim 15, characterised in that the control receptor is the interleukin-3 receptor.

17. Process according to one of claims 1 to 13, characterised in that, in addition, mammalian cells which are transformed with the recombinant DNA defined in a) and do not express a functional interleukin-5 receptor are incubated under identical conditions with the substance to be investigated and the concentration of the reporter gene product is measured.

18. Process according to one of claims 1 to 17, characterised in that it is used as a screening assay, in which the test substance is one of a number of substances with which a predetermined number of mammalian cells are incubated under predetermined conditions, the concentration of the reporter gene product is measured and the selectivity-of the substances for the interleukin-5 dependent signal transduction pathway is checked.

19. Mammalian cells, characterised in that a) they are transformed with a recombinant DNA containing a reporter gene and a regulatory sequence which responds to a change in concentration of one or more secondary messenger substances of an interleukin-5 receptor-dependent signal transduction pathway, and in that b) they are transformed with recombinant DNA, coding for the α-subunit or for the α- and β-subunits of the interleukin-5 receptor, so that they express the functional interleukin-5 receptor.

20. Mammalian cells according to claim 19, characterised in that they are human cells.

21. Mammalian cells according to claim 19, characterised in that they are murine cells.

22. Mammalian cells according to one of claims 19 to 21, characterised in that they naturally express only the β-subunit of the interleukin-5 receptor and are transformed with recombinant DNA containing the sequence which codes for the α-subunit.

23. Mammalian cells according to claim 22, characterised in that the α-subunit originates from another species.

24. Human cells according to claim 22, characterised in that they are transformed with recombinant DNA which contains the sequence coding for the human α-subunit.

25. Murine cells according to claim 22, characterised in that they are transformed with recombinant DNA which contains the sequence coding for the murine α-subunit.

26. Mammalian cells according to one of claims 19 to 25, characterised in that the recombinant DNA defined in a) contains the promoter of the c-fos-gene as a regulatory sequence.

27. Mammalian cells according to one of claims 19 to 25, characterised in that the recombinant DNA defined in a) contains the promoter of the ICAM-1-gene as a regulatory sequence.

28. Mammalian cells according to one of claims 19 to 27, characterised in that the recombinant DNA defined in a) contains the luciferase gene as reporter gene.

## Revendications

1. Procédé de détermination de l'effet modulateur d'une substance sur une voie de transmission de signaux dépendant d'un récepteur dans la cellule humaine ou animale, caractérisé en ce que l'on détermine l'effet modulateur de la substance sur un composant dans la voie de transmission de signaux qui est déclenché par activation du récepteur à l'interleukine 5, en incubant avec la substance à étudier des cellules de mammifère qui
a) sont transformées avec un ADN recombiné contenant un gène reporter et une séquence régulatrice qui réagit à une variation de concentration d'un ou plusieurs seconds messagers de la voie de transmission de signaux dépendant du récepteur à l'interleukine 5 de sorte que l'expression du gène reporter est modulée par une variation de concentration du messager et qui, en outre,
b) expriment le récepteur à l'interleukine 5 fonctionnel, en mesurant la concentration du produit du gène reporter et en examinant si l'effet de la substance à tester mis en évidence est sélectif à l'égard de la voie de transmission de signaux dépendant du récepteur à l'interleukine 5, par le fait que l'on applique en outre la substance, dans des conditions identiques, sur des cellules de mammifère témoins qui expriment un récepteur témoin différent du récepteur à l'interleukine 5 et qui sont transformées avec un ADN recombiné qui contient le gène reporter et une séquence régulatrice qui réagit à la variation de concentration d'un second messager de la voie de transmission de signaux dépendant du récepteur témoin, et en comparant la concentration du produit du gène reporter dans les cellules exprimant le récepteur à l'interleukine 5 avec la concentration dans les cellules témoins.

2. Procédé selon la revendication 1 caractérisé en ce que l'on étudie la substance à tester concernant son effet antagoniste pour la voie de transmission de signaux dépendant du récepteur à l'interleukine 5 en appliquant en outre de l'interleukine 5 sur les cellules.

3. Procédé selon la revendication 1 caractérisé en ce que l'on utilise des cellules qui expriment naturellement le récepteur à l'interleukine 5 fonctionnel.

4. Procédé selon la revendication 1 caractérisé en ce que l'on utilise des cellules qui n'expriment naturellement que la sous-unité β du récepteur à l'interleukine 5 et qui sont transformées avec un ADN recombiné qui contient la séquence codant la sous-unité α du récepteur à l'interleukine 5 de sorte que les cellules expriment le récepteur à l'interleukine 5 fonctionnel.

5. Procédé selon la revendication 1 caractérisé en ce que l'on utilise des cellules qui sont transformées avec des ADN recombinés qui contiennent les séquences codant la sous-unité α et les séquences codant la sous-unité β du récepteur à l'interleukine 5 de sorte qu'elles expriment le récepteur à l'interleukine 5 fonctionnel.

6. Procédé selon la revendication 5 caractérisé en ce que les séquences d'ADN codant la sous-unité a et les séquences d'ADN codant la sous-unité β sont présentes sur deux plasmides séparés.

7. Procédé selon l'une des revendications 1 à 6 caractérisé en ce que les cellules expriment un récepteur à l'interleukine 5 humain fonctionnel.

8. Procédé selon l'une des revendications 1 à 6 caractérisé en ce que les cellules expriment un récepteur à l'interleukine 5 murin fonctionnel.

9. Procédé selon l'une des revendications 1 à 6 caractérisé en ce que les cellules expriment un récepteur à l'interleukine 5 fonctionnel dont la sous-unité a et la sous-unité β proviennent d'espèces différentes.

10. Procédé selon la revendication 9 caractérisé en ce que la sous-unité α est d'origine humaine.

11. Procédé selon l'une des revendications 1 à 10 caractérisé en ce que l'ADN recombiné défini en a) contient le promoteur du gène c-fos comme séquence régulatrice.

12. Procédé selon l'une des revendications 1 à 10 caractérisé en ce que l'ADN recombiné défini en a) contient le promoteur du gène d'ICAM-1 comme séquence régulatrice.

13. Procédé selon la revendication 11 ou 12 caractérisé en ce que l'ADN recombiné défini en a) contient un gène de luciférase comme gène reporter.

14. Procédé selon l'une des revendications 2 à 13 caractérisé en ce que l'on étudie la substance à tester en ce qui concerne son -effet antagoniste en incubant en outre des cellules qui expriment, outre des récepteurs pour d'autres facteurs de croissance, le récepteur à l'interleukine 5 fonctionnel et qui sont dépendantes dans leur croissance de la présence d'au moins le ligand pour l'un de ces récepteurs, d'une part avec l'interleukine 5 seule et d'autre part avec l'interleukine 5 et la substance à tester et on compare la prolifération des deux cultures de cellules.

15. Procédé selon l'une des revendications 1 à 13 caractérisé en ce que l'on examine la sélectivité de l'effet modulateur de la substance sur la voie de transmission de signaux dépendant du récepteur à l'interleukine 5 en appliquant en outre la substance dans des conditions identiques sur des cellules de mammifère témoins qui expriment un récepteur témoin différent du récepteur à l'interleukine 5 et qui sont transformées avec un ADN recombiné défini en a) dont la séquence régulatrice réagit à une variation de concentration d'un ou plusieurs seconds messagers non seulement de la voie de transmission de signaux dépendant du récepteur à l'interleukine 5 mais aussi de la voie de transmission de signaux dépendant du récepteur témoin, et en ce que l'on compare la concentration du produit du gène reporter dans les cellules exprimant le récepteur à l'interleukine 5 avec la concentration dans les cellules témoins.

16. Procédé selon la revendication 15 caractérisé en ce que le récepteur témoin est le récepteur à l'interleukine 3.

17. Procédé selon l'une des revendications 1 à 13 caractérisé en ce que l'on incube en outre des cellules de mammifère qui sont transformées avec l'ADN recombiné défini en a) et qui n'expriment aucun récepteur à l'interleukine 5 fonctionnel dans des conditions identiques avec la substance à étudier et on mesure la concentration du produit du gène reporter.

18. Procédé selon l'une des revendications 1 à 17 caractérisé en ce qu'il est utilisé comme test de sélection par le fait que la substance à étudier est une substance parmi de nombreuses substances avec lesquelles on incube un nombre prédéterminé de cellules de mammifère dans des conditions prédéterminées, on mesure la concentration du produit du gène reporter et on examine la sélectivité des substances à l'égard de la voie de transmission de signaux dépendant de l'interleukine 5.

19. Cellules de mammifère caractérisées en ce que a) elles sont transformées avec un ADN recombiné contenant un gène reporter et une séquence régulatrice qui réagit à une variation de concentration d'un ou plusieurs seconds messagers d'une voie de transmission de signaux dépendant du récepteur à l'interleukine 5 et en ce que b) elles sont transformées avec un ADN recombiné codant la sous-unité a ou la sous-unité α et la sous-unité β du récepteur à l'interleukine 5 de sorte qu'elles expriment le récepteur à l'interleukine 5 fonctionnel.

20. Cellules de mammifère selon la revendication 19 caractérisées en ce que ce sont des cellules humaines.

21. Cellules de mammifère selon la revendication 19 caractérisées en ce que ce sont des cellules murines.

22. Cellules de mammifère selon l'une des revendications 19 à 21 caractérisées en ce qu'elles n'expriment naturellement que la sous-unité β du récepteur à l'interleukine 5 et en ce qu'elles sont transformées avec un ADN recombiné qui contient la séquence codant la sous-unité α.

23. Cellules de mammifère selon la revendication 22 caractérisées en ce que la sous-unité a provient d'une autre espèce.

24. Cellules humaines selon la revendication 22 caractérisées en ce qu'elles sont transformées avec un ADN recombiné qui contient la séquence codant la sous-unité a humaine.

25. Cellules murines selon la revendication 22 caractérisées en ce qu'elles sont transformées avec un ADN recombiné qui contient la séquence codant la sous-unité a murine.

26. Cellules de mammifère selon l'une des revendications 19 à 25 caractérisées en ce que l'ADN recombiné défini en a) contient le promoteur du gène c-fos comme séquence régulatrice.

27. Cellules de mammifère selon l'une des revendications 19 à 25 caractérisées en ce que l'ADN recombiné défini en a) contient le promoteur du gène d'ICAM-1 comme séquence régulatrice.

28. Cellules de mammifère selon l'une des revendications 19 à 27 caractérisées en ce que l'ADN recombiné défini en a) contient le gène de la luciférase comme gène reporter.
